# EUROPEAN PATENT APPLICATION

(11) **EP 1 533 616 A1**
(43) Date of publication of application: **25.05.2005**
(21) Application number: 03736119.3
(22) Date of filing: 09.06.2003
(51) Int. Cl.: G01N 33/53, C07K 16/18

(54) **DETECTION KIT, ASSAY PLATE TO BE USED THEREIN, DETECTION METHOD, EVALUATION METHOD, POLYCLONAL ANTIBODY OF FROG VITELLOGENIN AND PROCSS FOR PRODUCING THE SAME**

(30) Priority: 07.06.2002 JP 2002167920
(71) Applicant: Towa Kagaku Co., Ltd., Hiroshima-shi, Hiroshima 730-0841 (JP); Japan Envirochemicals, Ltd., Osaka-shi, Osaka 541-0045 (JP)
(72) Inventor: KAWAHARA, Akira, Higashihiroshima-shi, Hiroshima 739-2121 (JP); GODA, Yasuhiro JAPAN ENVIROCHEMICALS, LTD., Osaka-shi, Osaka 532-0024 (JP); MITSUI, Naoko TOWA KAGAKU CO., LTD., Hiroshima-shi, Hiroshima 730-0841 (JP)
(74) Representative: HOFFMANN - EITLE
(86) International application number: PCT/JP2003/007296
(87) International publication number: WO 2003/104806

(57) **Abstract**

On a surface of a well previously disposed on a plate, a primary antibody that recognizes vitellogenin is solid-phased, in the well a sample obtained from a test body exposed to an environment is injected to react, followed by injecting a secondary antibody that is labeled with an enzyme and recognizes the vitellogenin, further followed by injecting a chromogenic reagent to cause a coloring reaction and by measuring the stained amount, still further followed by calculating an amount of vitellogenin from the stained amount to evaluate an environment based on the amount of vitellogenin.

## Description

### Field of the invention

The present invention relates to a technical field that evaluates an environment with, for instance, frog vitellogenin. In addition, the present invention relates to, in particular, a detection kit of frog vitellogenin, a measurement plate, a method of detecting vitellogenin, an evaluation method and polyclonal antibodies to frog vitellogenin.

### Background of the invention

Recently, influences of various chemical substances on living things including humans and ecosystems are clearly existed.

Among these, influences of endocrine disruptors that are generally called as environmental hormones, disrupt an endocrine system of a living thing and disturb the homeostasis thereof are becoming serious.

It is considered that the endocrine disruptor (hereinafter, referred to also as environmental hormone) generates actions similar to hormones that a living thing originally has, or disturbs the actions, and thereby causes the abnormality to the living thing. As a working point of the endocrine disruptor, various phases such as binding with a hormone receptor, binding with a hormone (ligand), synthesis of hormones in a living thing and hormone metabolism are pointed out; however, since action mechanisms of the endocrine disruptors on living things are diversified, at present, the mechanism of the endocrine disrupting actions due to the endocrine disruptors are not yet clearly understood.

Influences of the endocrine disruptors on the living things have been confirmed mainly with the malformation and the behavior disorder as an indicator. However, when the malformation or the behavior disorder is taken as an indicator, these are quantified with difficulty and both the sensitivity and the accuracy thereof are low; accordingly, it is very risky and difficult to estimate the actions of the chemicals from the malformation or the behavior disorder. In this connection, a molecular marker that can quantitatively evaluate, before the abnormality in the phenotype of living things such as the malformation or the behavior disorder are generated, the actions of the chemicals or the environment on living things is in demand.

As one of the molecular markers that are provided with such characteristics, vitellogenin that are yolk protein precursors of an egg-laying animal are gathering attention. Vitellogenin is normally actively synthesized in breeding times in a liver of a female individual; on the contrary, the vitellogenin is not detected or present originally in male blood. From these characteristics, vitellogenin is gathering attention as a marker that can evaluate with the sensitivity and the quantitativity the endocrine disrupting action of the chemicals or environment.

In order to detect vitellogenin, there are descriptions in, for instance, JP-A Nos. 2001-218582, 2001-122899, and 2000-125867, on methods of detecting vitellogenin of fishes such as carps and Oryzias latipes, and in actuality detection kits of vitellogenin is practically used.

### Disclosure of Invention

However, in order to understand the influences of the chemicals on an entire ecosystem and cope with these, the influences of the chemicals on not only fishes but also living things located on various nutrition stages and phyletic evolution stages have to be properly investigated and evaluated.

Furthermore, a structure of the vitellogenin is very complicated and rather diversified depending on the species; accordingly, it is very difficult to measure vitellogenin due to other species with an existing detection kit that is constituted for a particular species.

The present invention was achieved in view of the above situations and intends to provide a technology that can detect vitellogenin of amphibians, in particular, frogs with the quantitativity and sensitivity to precisely evaluate the chemicals and the environment.

The present inventors studied hard and achieved the above object by means shown below.

That is, a detection kit of frog vitellogenin according to the invention includes a measurement plate having a plate body that has a bottomed well wherein a sample is injected and primary antibodies that are solid-phased on a surface of the well and recognizes the frog vitellogenin; standard frog vitellogenin that are injected in the well where the primary antibodies are solid-phased; and secondary antibodies that are injected in the well where the sample or standard is injected to recognize the frog vitellogenin.

Furthermore, according to a separate viewpoint, a detection kit according to another aspect of the invention includes a plate body that has a bottomed well wherein a sample is injected; primary antibodies that are solid-phased on a surface of the well and recognizes the frog vitellogenin; standard frog vitellogenin that are injected in the well where the primary antibodies are solid-phased; and secondary antibodies that are injected in the well where the sample or the standard is injected to recognize the frog vitellogenin.

Here, the environment means the chemicals present in the environment or the environment that is polluted with the chemicals.

Furthermore, the sample here is a blood plasma or blood serum, a tissue and a cell of a frog. Examples of the frog that can be used in the invention include Rana japonica, Rana nigoromaculata, Rana rugosa, Microhyla ornate, Bombina bombina, Xenopuas laevis, and Xenopus tropicalis. Among these, when the Xenopus laevis is used, irrespective of seasons, a large amount of eggs, resultantly, adults can be obtained and individuals can be easily maintained. Accordingly, it can be preferably used.

In the invention, according to the configuration as mentioned above, the frog vitellogenin can be speedily and easily detected.

In the invention, in particular, primary antibodies that recognize the frog vitellogenin is solid-phased on a surface of a well of a plate; accordingly, according to, for instance, sandwich enzyme-linked immunosorbent assay (ELISA) involving a detection method according to the invention described below, vitellogenin can be detected.

An existing vitellogenin detection kit is for use in fishes alone; however, according to the invention, since vitellogenins of the amphibians including frogs can be measured with accuracy, evaluation of the chemicals and so on in these species can be properly carried out.

Furthermore, frogs are egg-lying animals and accumulate a large amount of yolk proteins in an egg in order to secure nutrition in the course of evolution. The vitellogenin is a precursor of the yolk protein and normally synthesized in a liver of a female individual; namely, it is not synthesized in a liver of a male individual. However, in the case of a male individual exposed to estrogen and so on, vitellogenin that is not originally synthesized are synthesized in a liver. Accordingly, vitellogenin, even when the endocrine disrupting action mechanism due to the endocrine disruptors is not clear, are effective as a marker that can evaluate the endocrine disrupting properties in the environment with the sensitivity.

Still furthermore, since a life circle of frogs covers an amphibian environment, frogs are characteristic in that they are exposed not only to chemicals in water sites such as rivers, lakes, and underground waters or environment soils but also to chemicals in air. Accordingly, when influences of the chemicals such as the environmental hormones on wildlife are evaluated, the frogs can be advantageously used.

Furthermore, the sample is blood plasma or blood serum of a frog.

As a sample that can be used in the detection kit according to the invention, samples due to blood plasma or blood serum or a certain kind of tissues or cells of a frog that was exposed to the wildlife or the environment for a definite period, or of a frog that was exposed to the chemicals in a laboratory can be used.

When these are used as samples, the detection precision can be improved.

In the plate, a plurality of wells can be preferably disposed and various known plates can be used. Owing to the presence of the plurality of wells, various kinds of samples can be simultaneously processed, resulting in an improvement in the processing efficiency.

In addition, since antibodies are solid-phased on a surface of each of the plurality of wells, when the sample is dispensed, an antigen-antibody reaction can be readily caused, resulting in a rapid processing.

In the invention, the secondary antibody is covalently coupled with a labeling compound.

Here, the labeling compound means enzymes such as HRP (horseradish peroxidase), biotin and so on.

Thereby, when the enzyme-labeled secondary antibodies or the secondary antibodies and the third enzyme-labeled detection compounds that recognize the secondary antibodies are injected to the plate followed by reacting with a chromogenic reagent, the labeling enzymes develop a color, and when an amount of stained is measured in terms of the absorbance, an amount of vitellogenin can be quantified.

The primary antibodies are adsorbed and solid-phased on a surface of the well and blocked by a blocking reagent.

Thereby, the solid-phased primary antibodies are assuredly fixed, resulting in an improvement in the reliability of detection results.

Furthermore, a detection kit involving still another aspect of the invention includes a first plate that has a bottomed well in which a sample and antibodies that recognize frog vitellogenin and are conjugated with a labeling compound are injected and mixed; a second plate body that has a bottomed well in which a mixture liquid of the sample and antibodies is injected; and standard frog vitellogenin that are solid-phased as antigens on a surface of the well of the second plate.

Since according to the configuration like this, the detection kit becomes one that is particularly suitable for an competitive method among enzyme-linked immunosorbent assay, the processing can be efficiently carried out according to the method and proper results can be obtained.

Furthermore, the sample is preferable to be blood plasma or blood serum of a frog. Thereby, vitellogenin can be efficiently detected.

Still furthermore, the antigens are adsorbed and solid-phased on a surface of the well and blocked by a blocking reagent.

Thereby, nonspecific antibodies are excluded from binding; accordingly, the reliability of evaluation also can be improved.

The measurement plate according to the invention includes a plate body having bottomed wells wherein a sample is injected; and primary antibodies that are solid-phased on a surface of the well and recognize the frog vitellogenin.

According to the configuration like this, when it is used as a measurement plate of a detection kit involving a sandwich method, the frog vitellogenin can be efficiently detected.

That is, since antibodies are solid-phased beforehand on a surface of the plate, only by dispensing a sample there, speedily, an antigen-antibody reaction can be obtained. Furthermore, since there is no need of antibodies being solid-phased every time when the process is applied, results can be inhibited from scattering.

Furthermore, a measurement plate involving another aspect according to the invention includes a plate body that has bottomed wells in each of which a mixture of a sample and antibodies that recognize frog vitellogenin and are labeled with a labeling compound is injected; and frog vitellogenin that are solid-phased as antigens on a surface of each of the wells of the plate.

According to the configuration like this, since in this case antigens are solid-phased on a surface of the plate, it can be used as a measurement plate of a detection kit involving competitive binding assay antagonism, thereby frog vitellogenin can be efficiently detected.

The detection method according to the invention detects the frog vitellogenin with the abovementioned detection kit.

According to the configuration like this, since a detection kit excellent in the sensitivity can be used, the reliability in the detection can be improved.

Furthermore, a detection method of the frog vitellogenin involving another aspect includes reacting a sample and antibodies that recognize vitellogenin contained in the sample; and reacting a complex of the vitellogenin contained in the sample and the antibody with a secondary antibody that is labeled with a labeling compound and recognizes the vitellogenin.

According to the configuration like this, since vitellogenin is detected by means the enzyme-linked immunosorbent assay, the detection accuracy can be improved and a processing time can be shortened. In particular, the detection method according to the invention is suitable for the sandwich method and can improve the detection accuracy of vitellogenin.

Furthermore, reacting the secondary antibody in the complex with a chromogenic reagent and measuring an amount of vitellogenin in the sample based on the coloring reaction is further included.

Thereby, the labeled vitellogenin can be assuredly quantified according to the sandwich method.

Furthermore, still another aspect involving the detection method of the frog vitellogenin according to the invention includes reacting a sample and antibodies that are labeled with an enzyme and recognize vitellogenin contained in the sample to obtain a complex; and competitively reacting the complex and the vitellogenin.

The detection method involving the aspect is an competitive method, and according to the configuration like this, the detection sensitivity of vitellogenin can be improved in accordance with a shape of the sample.

Furthermore, the detection method further includes reacting a reaction product obtained owing to the competitive reaction and a chromogenic reagent and measuring an amount of vitellogenin in the sample based on a coloring reaction.

Thereby, vitellogenin detected owing to the competitive reaction can be assuredly quantified.

Thus, the detection method according to the invention is a so-called enzyme-linked immunosorbent assay (ELISA method) and can be preferably applied to both the sandwich method and the competitive method.

Thereby, even when various samples are used, the enzyme-linked immunosorbent assay excellent in the sensitivity in accordance with a sample shape can be used.

Furthermore, an antibody that is used in the detection method may be any one of a polyclonal antibody and a monoclonal antibody.

Still furthermore, the sample is preferably blood plasma or blood serum of a frog. Thereby, the detection sensitivity can be improved.

An evaluation method according to the invention includes reacting a sample and antibodies that recognize vitellogenin contained in the sample; reacting secondary antibodies that are labeled with a labeling compound and recognize the vitellogenin to complexes of vitellogenin contained in the sample and the antibodies; reacting labeling enzymes in the secondary antibodies bonded to the complexes and a chromogenic reagent to measure a stained amount; and calculating an amount of vitellogenin from the stained amount and evaluating an environment based on the amount of vitellogenin.

According to the configuration like this, based on the sandwich method, accurate environmental evaluation can be carried out.

Furthermore, the sample is preferably blood plasma or blood serum of a frog.

Still furthermore, an evaluation method of another aspect involving the invention includes reacting a sample and antibodies that are labeled with a labeling compound and recognize vitellogenin contained in the sample to obtain complexes; causing the complexes and vitellogenin to competitively react; and reacting reaction products obtained owing to the competitive reaction and a chromogenic reagent, calculating an amount of vitellogenins based on the coloring reaction to evaluate an environment based on the amount of vitellogenins.

Also in the aspect, the sample is preferably blood plasma or blood serum of a frog.

According to the configuration like this, based on the competitive method, accurate environmental evaluation can be performed.

Furthermore, as mentioned above, in the evaluation method according to the invention, both the sandwich method and the competitive method can be appropriately applied; accordingly, in accordance with various sample shapes, evaluations can be performed.

Polyclonal antibodies of frog vitellogenin according to the invention can be obtained in such a manner that a mammal is immunized with frog vitellogenin as an antigen, antiserum are sampled from the immunized mammal, and IgG are isolated from the antiserum.

When thus obtained antibodies are used, the frog vitellogenin can be detected specifically and with sensitivity, resulting in properly evaluating the environment.

In a manufacturing method according to the invention of polyclonal antibodies, IgG obtained from antiserum that are sampled from a mammal immunized with frog vitellogenin as an antigen are purified by use of an affinity column, and thereby polyclonal antibodies can be obtained.

Furthermore, the affinity column is bonded with male frog serum proteins.

Still furthermore, the affinity column is bonded with frog vitellogenin.

When thus configured, since binding specificity of the antibodies as that can be improved, thereby the accuracy in the detection and evaluation method can be improved as well.

An evaluation method involving another aspect of the invention includes cultivating hepatocytes of an amphibian; administering a sample chemical to the hepatocytes; and detecting a response to a sample chemical of the cultivated hepatocytes.

Here, as the hepatocytes used here, ones from amphibians including adults and larvae can be used.

Furthermore, the evaluation samples here indicate various kinds of chemicals, and environmental samples such as river water, factory wastewater, water processed at sewage plants or extracted components from soil.

According to the configuration like this, with hepatocytes, to a lot of samples, the response of the hepatocytes can be directly detected economically, speedily, conveniently, and without being influenced by hormones in living things, and a strict exposure condition also can be set.

A detection method involving still another aspect of the invention detects vitellogenin by use of the abovementioned detection kit.

Here, the response mainly indicates induction of vitellogenin synthesis. However, other than this, with induction of transferrin, albumin, and so on as a marker, actions of the chemicals belonging to other environmental hormones can be variously evaluated.

### Brief Description of the Drawings

Fig. 1 is a schematic view showing an entirety of a detection kit according to the present invention.
Fig. 2 is a schematic view of an entirety of a measurement plate of a detection kit according to the invention.
Fig. 3 is a partial sectional view of a well of a measurement plate involving a sandwich method of a detection kit according to the invention.
Fig. 4 is a partial sectional view of a well of a measurement plate involving an competitive method of a detection kit according to the invention.
Fig. 5 is a process chart explaining an competitive method in a detection method according to the invention.
Fig. 6 is a process chart explaining a sandwich method in a detection method according to the invention.
Figs. 7A and 7B are images showing the specificity of a vitellogenin antibody according to the invention.
Figs. 8A and 8B are graphs showing the sensitivity in detecting vitellogenin in detection methods according to the invention.
Fig. 9 shows a graph and an image showing detection of vitellogenin in a western blotting as a comparative example.
Fig. 10 shows graphs showing induction of vitellogenin synthesis in exposure in water according to an environmental evaluation method according to the invention.
Fig. 11 shows graphs showing induction of vitellogenin synthesis in direct injection according to an environmental evaluation method according to the invention.

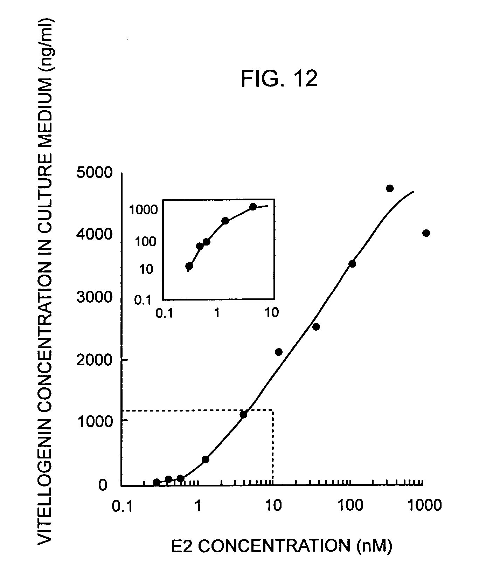

Fig. 12 is a graph showing induction of vitellogenin synthesis in which cultivated hepatocytes are used.
Fig. 13 is an explanatory diagram of a method for afinity-purified polyclonal antibodies.
Fig. 14 shows an isolating purification chart pattern of VTGs owing to anion exchange chromatography (upper one) and a photograph SDS-PAGE/CBB-staining images of a fraction solution (lower one).
Fig. 15 shows examples of concentration quantification of a VTG standard.
Fig. 16 is a diagram showing a gel-stained pattern of a VTG standard.
Fig. 17 shows a diagram showing results of confirmation of concentrations of VTG standards according to an ELISA method.
Fig. 18 is a diagram showing a rise in specific antibody titers after affinity purification.
Fig. 19 is a diagram showing comparison of specific antibody titers between affinity-purified polyclonal antibodies of different immunization lots.
Figs. 20A and 20B are diagrams showing the specificity of antibodies against vitellogenin, 20A showing a CBB-stained pattern of a blood plasmaample, 20B showing western blotting.
Fig. 21 is a diagram showing results of study on conditions for maximizing quantity of concentrations of solid-phased antibodies.
Fig. 22 is a diagram showing results of study on conditions for maximizing detection level by use of HRP labeled antibodies.
Figs. 23A through 23D are diagrams showing addition/recovery rate experiments and studies on BSA addition for improving the recovery rate, 23A showing a case where the BSA is not included, 23B showing a case where 0.5 percent of BSA is included, 23C showing a case where 1 percent of BSA is included, 23D showing calibration curves.
Fig. 24 is a diagram showing the linear regression curves in the sample dilution experiments after a sample dilution liquid is optimized.
Fig. 25A is a diagram showing a situation where a frog is wrapped with tissue paper.
Fig. 25B is a diagram showing a situation where a frog is stabbed at a flank with a needle.
Fig. 25C is a diagram showing a situation where blood is flowing out in ball from a flank of a frog.
Fig. 25D is a diagram showing a situation where blood is being sampled from a frog.
Fig. 26 is a diagram showing an ELISA KIT calibration curve.
Fig. 27 is a diagram showing numerical values characterizing the ELISA KIT calibration curve.
Fig. 28 is a diagram showing an ELISA calibration curve obtained by use of biotinylated antibodies.
Figs. 29A and 29B are diagrams showing the cross-reactivity of antibody to VTGs of other species of frogs, 29A showing a case of ELISA, 29B showing a case of SDS-PAGE/CBB staining.
Fig. 30 is a diagram showing VTG synthesis induction in hepatocyte in primary culture.
Fig. 31 is a diagram showing a antagonistic effect on VTG synthesis in hepatocyte in primary culture.
Fig. 32 is a diagram showing a vitellogenin (VTG) calibration curve (part 1).
Fig. 33 is a diagram showing a vitellogenin recovery rate curve (part 1).
Fig. 34 is a diagram showing a vitellogenin (VTG) calibration curve (part 2).
Fig. 35 is a diagram showing a linear decline of the response due to the vitellogenin dilution-dependent linear regression (part 2).
Fig. 36 is an exploded diagram of an immunochromatography device involving application examples according to the invention.

### Best Mode for Carrying Out the Invention

In what follows, embodiments according to the present invention will be explained with reference to the drawings.

Firstly, a detection kit will be explained. The detection kit is one that can accurately detect frog vitellogenin by enzyme-linked immunosorbent assay (ELISA) and can cope with both a sandwich method and an competitive method of the ELISA.

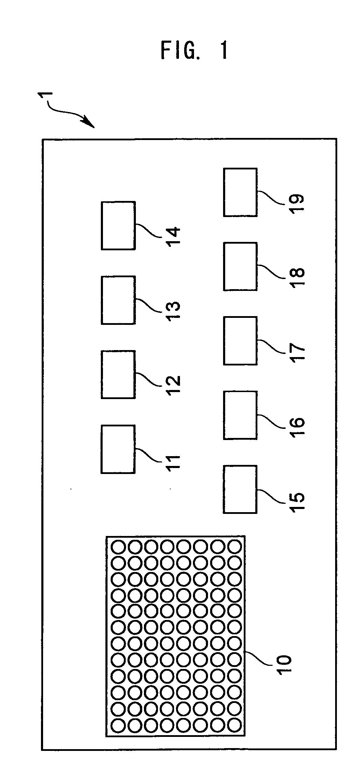

Firstly, a detection kit that detects frog vitellogenin by means of a sandwich ELISA will be explained based on the drawings. Fig. 1 is a diagram schematically showing a configuration of the whole of a vitellogenin detection kit involving the present embodiment.

Here, a vitellogenin detection kit 1 includes a plate 10 having bottomed wells 101 in each of which a primary antibody 12 that recognize vitellogenin is solid-phased and therein a sample is injected; reference frog vitellogenin 11 as a specimen; and secondary antibodies 13 labeled with an enzyme or a biotin.

Furthermore, other than these, a test body dilution liquid 14 for diluting a sample, an antibody dilution liquid 15 for diluting antibodies, a base solution and a chromogenic reagent 16 for developing color due to labels of the secondary antibodies 13, a buffer solution 17 for stabilizing a liquid in process, a reaction stop liquid 18 for suppressing an excessive reaction or a cleaning liquid 19 for washing the plate at a predetermined time, and a reagent normally used in the ELISA method.

In the detection kit 1, according to the configuration like this, into the wells 101 of the plate 10, samples prepared from frogs exposed to an environment are injected, an antigen-antibody reaction between vitellogenin contained in the sample or the specimen 11 and the primary antibodies 12 or secondary antibodies 13 is caused, followed by detecting it based on the ELISA method, and thereby vitellogenin can be detected with high sensitivity.

Here, as samples that are used in the detection kit 1, samples derived from body fluids or tissues or cells of a frog that is exposed to the wildlife or environment for a definite period, or a frog that is exposed to the chemicals in a laboratory can be used. As will be mentioned below, blood plasmas, a homogenate of liver, or hepatocytes in primary culture of a frog can be preferably used. Vitellogenin is synthesized in a liver and transferred by blood; accordingly, with these as samples, vitellogenin can be detected with accuracy.

Furthermore, the sample is preferable to be frog blood plasmas or blood serum. Still furthermore, the sample is preferable to be media of cultured hepatocytes.

In the case of the sample being the tissue, a liver homogenate can be preferably used, and in the case of it being the cell, (x laevis) hepatocytes in primary culture are preferable.

Thereby, the detection sensitivity of vitellogenin in a frog body can be improved.

Furthermore, these samples, the primary antibodies, the secondary antibodies and the specimen vitellogenin is diluted with the abovementioned buffer solution to process.

Still furthermore, as a label of the secondary antibody, horseradish peroxidase (HRP) or biotin is preferable; however, various other known labels can be used.

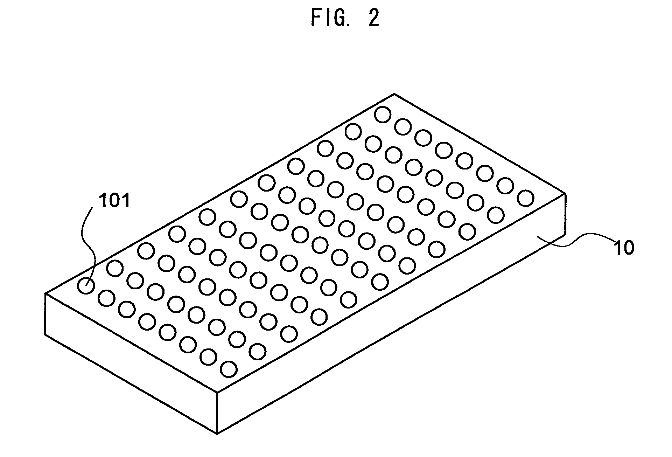

Fig. 2 is a schematic diagram of a plate 10 of the detection kit 1 involving the embodiment.

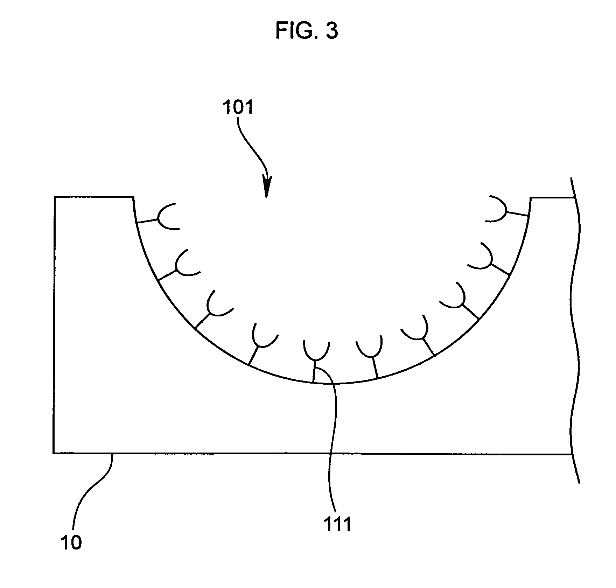

As shown in Fig. 2, on the plate 10 of the detection kit 1, there is disposed a plurality of wells 101 in which a sample is discharged. Here, 12 plates on each of which, for instance, 8 wells are disposed in one row are connected and thereby 96 wells are disposed, and in each of the wells 101 a series of processes is carried out. A partial sectional view of the well is shown in Fig. 3.

On a surface of the well 101, by use of a sandwich method of detection methods described below, antibodies are solid-phased beforehand. In Fig. 3, as an example, a state where primary antibodies 111 are solid-phased is shown.

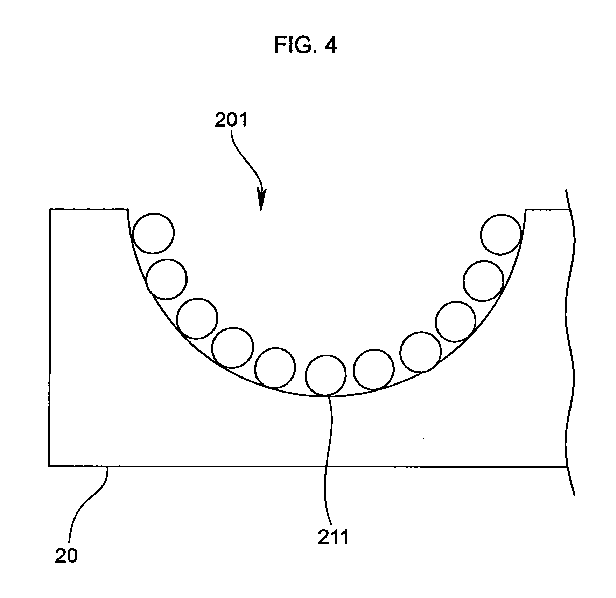

In the next place, a configuration of a detection kit that is used in an competitive method will be roughly explained. Reagents and so on that are used as a kit are substantially similar to those of the sandwich method. However, as shown in Fig. 4, in the competitive method, in a well 201 of a plate 20, as an antigen, frog vitellogenin 211 are solid-phased, in a separate plate (not shown in the drawing) a sample and labeled antibodies are beforehand mixed, a mixture thereof is injected in the plate 20 followed by measuring the reaction, and thereby vitellogenin can be detected.

Thus, when the detection kit involving the embodiment is used, even when a concentration of vitellogenin contained in the sample is low, it can be detected with sensitivity and thereby more refined environmental evaluation can be carried out. Furthermore, when antigens or antibodies are solid-phased beforehand, the convenience also can be improved, the processing can be sped up and the reliability of obtained results can be improved as well.

An existing kit for detecting vitellogenin can be applied only to the detection of vitellogenin of Oryzias latipes or carps and cannot cope with higher animals having a somatic system higher than the fishes. However, according to the inventive kit, vitellogenin of the amphibians, frogs, can be detected, and thereby over-all environmental evaluation can be performed.

In the next place, a detection method of frog vitellogenin will be explained.

In a detection method involving the embodiment the abovementioned detection kit is used, and, based on the enzyme immunoassay, that is, the ELISA method, vitellogenin is detected.

Now, in the ELISA method, two methods of a sandwich method and an competitive method are known. However, the detection method can cope with both.

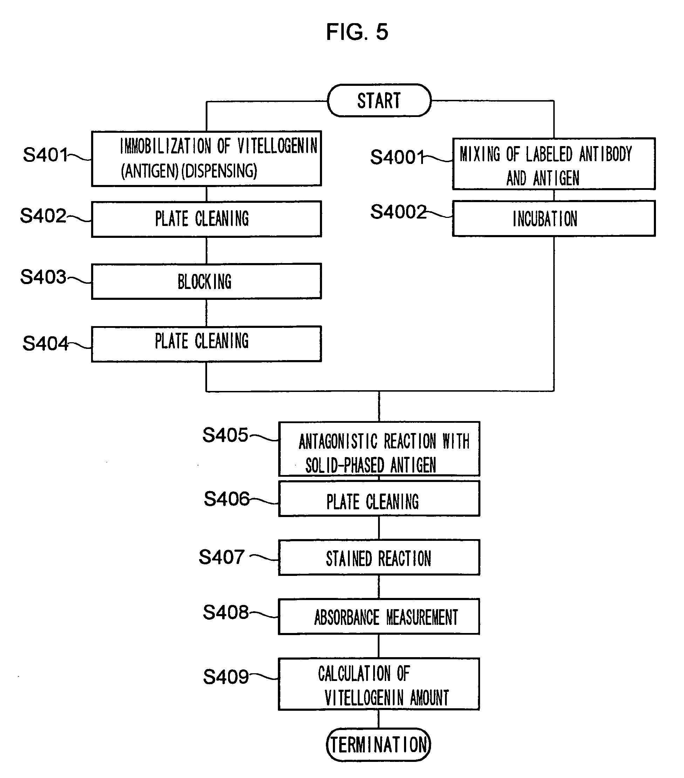

In what follows, processes in the respective methods will be explained with reference to the drawings. Fig. 4 is a process chart for explaining steps of the detection method according to the competitive method. Fig. 5 is a process chart for explaining steps of the detection method according to the sandwich method.

### Competitive method

Firstly, as solid-phased antigens, a preparation is solid-phased. A preparation vitellogenin is diluted, followed by dispensing on a micro-plate, further followed by incubating (step 401), still further followed by immobilizing. After a predetermined time has passed, the plate is washed (step 402) and a blocking reagent is dispensed to apply the blocking on a surface of a well of the plate (step 403). Thereafter, the plate is washed to remove an excessive chemical (step 404).

In parallel with the immobilization, on a separate plate, HRP labeled antibodies and a sample (or antigens) derived from a frog exposed to an environment are mixed (step 4001), followed by incubating (step 4002). Thus obtained antigen-antibody complexes are dispensed on a antigen-solid-phased plate to cause an competitive reaction (step 405). Subsequently, the plate is washed (step 406) followed by injecting a chromogenic substrate to cause a coloring reaction (step 407). When the reaction comes to completion, the absorbance is measured with a microreader or the like (step 408) and from a value thereof an amount of vitellogenin is calculated (step 409). Sandwich method

A diluted solution of primary antibodies is dispensed in each of wells of a micro-plate followed by incubating for a predetermined period (step 501). Thereafter, the plate is washed (step 502), a blocking reagent is dispensed to complete immobilization (step 503), followed by dispensing a sample derived from frogs in an environment or a preparation vitellogenin (diluted solution) to cause reacting (step 504). Thereafter, the plate is washed (step 505), followed by dispensing secondary antibodies to react (step 506). Subsequently, the plate is washed (step 507) followed by dispensing a chromogenic substrate to cause a coloring reaction (step 508). After the coloring reaction, the absorbance is measured with a microreader or the like (step 509) and from a result thereof an amount of vitellogenin is calculated (step 510).

As mentioned above, according to the detection method, two types of detection method can be used and various samples can be used. Furthermore, thereby, the detection sensitivity as well can be improved.

In the next place, an environmental evaluation method will be explained.

An environmental evaluation method involving the embodiment, by use of the abovementioned detection kit and the detection method, enables to use frog vitellogenin that has not been detected. Thereby, influences of the endocrine disruptors such as environmental hormones and various kinds of chemicals in the environments can be evaluated.

As a vitellogenin detection kit that is put into practical use at present, there is only one that can cope only with the fishes such as Oryzias latipes and carps. Accordingly, there is an inconvenience in that a comprehensive environmental evaluation including living organisms other than the fishes cannot be carried out. However, according to the environmental evaluation method according to the invention, owing to the use of frogs, there is an advantage in that based on an amount of vitellogenin detected from a frog, the environmental evaluation can be comprehensively performed.

Now, as a method of exposing a frog that is used as a sample to an environment, methods such as addition of a target substance to breeding water and direct injection to individuals can be used. Furthermore, by capturing a frog in wildlife and obtaining a sample therefrom, the natural environment as that can be easily and accurately evaluated; accordingly, evaluations of various environments can be performed.

In the environmental evaluation method involving the embodiment, samples are prepared from each of a thus obtained test body and a test body bred in a reference area, an amount of vitellogenin in the test body is measured and compared with that of the test body in the reference area, and therefrom the endocrine disrupting action of the target substance and the toxicity of the chemicals can be judged. That is, a male frog is bred in the presence of a chemical material (such as bisphenol A and phthalic acid ester) that is a target of evaluation, and an amount of vitellogenins in blood plasma is measured with time and compared with that of vitellogenin in blood serum obtained from a frog in the reference area. As a result, when an amount of vitellogenins obtained from the amphibians in an experimental area is higher than that in the reference area, it can be judged that the chemical material that is a target of evaluation causes the endocrine disrupting influences. Alternatively, after a male frog is bred in the coexistence of estrogen that is a reference and a chemical substance that is a target of evaluation, blood or the like is sampled, vitellogenin contained in the sample are reacted with antibodies according to the invention followed by measuring an amount of vitellogenins, and from the magnitudes of obtained measurements the toxicity (such as the endocrine disrupting influences) of the chemical substance can be evaluated. That is, in the presence of the chemical substance that is a target of evaluation, a male frog is bred, and an amount of vitellogenins in blood serum is measured with time and compared with that obtained a frog bred in the reference area. As a result, when an amount of vitellogenins obtained from a frog in an experimental area is lower than that in the reference area, it can be judged that the chemical substance that is a target of evaluation causes the endocrine disrupting influences.

Furthermore, when a vitellogenin concentration when a frog is bred in the presence of a certain concentration of estrogen and a vitellogenin concentration when a frog is bred in the presence of a chemical substance that is a target of evaluation are compared, the intensity of the endocrine disruption effect of the chemical substance that is a target of evaluation can be evaluated relative to the intensity of estrogen. That is, when an amount of vitellogenins when a frog is bred in the presence of a certain concentration (such as 1.0 ppm) of estrogen is same as that when a frog is bred in the presence of a certain concentration (such as 0.1 ppm) of a chemical substance of which intensity of the endocrine disruption effect is not known, the endocrine disruption effect of the chemical substance can be judged as 10 times stronger than estrogen.

Furthermore, according to the evaluation method, when an amount of vitellogenins in a body liquid of a male frog living in a river or a lake that is a target of evaluation is measured, a situation of pollution of the environment owing to the chemical substance having the endocrine disruption effect can be evaluated.

In the next place, a polyclonal antibody of frog vitellogenin will be explained.

A polyclonal antibody of the frog vitellogenin can be manufactured according to any one of so far known methods (such as Sambrook, J et al., *Molecular Cloning*, Cold Spring Harbor Laboratory Press (1989)). However, an antibody manufactured according to a manufacturing method of polyclonal antibody described below is preferable. In what follows, based on the manufacturing method, the polyclonal antibody will be described.

To a mammal such as a rat, a mouse and a rabbit, frog vitellogenin is administered as an antigen to immunize. An amount of administration of the antigen per one animal is, when, for instance, an adjuvant is used, 500 to 1000 µg. As the adjuvant, Freund's complete adjuvant (FCA), Freund's incomplete adjuvant (FIA) and aluminum hydroxide adjuvant can be cited. The immunization is mainly performed by hypodermic injection. Furthermore, an interval of the immunization is not particularly restricted; however, it may be applied with an interval from several days to several weeks, and preferably after 3 weeks from a first immunization, 1 to 2 times at an interval of 2 weeks. After 5 to 20 days from the last immunization day, preferably after from 7 to 14 days, antiserum are sampled. Subsequently, from the obtained antiserum, by means of ammonium sulfate fractionation and DEAE-Sephadex column chromatography, IgG are obtained.

In order to obtain vitellogeninpecific antibodies, by use of an absorption column covalently coupled with frog serum proteins, adsorption purification is carried out, and by use of a frog vitellogenin column affinity purification is carried out.

Thus, not only IgG are isolated from the antiserum but also by applying the affinity purification, an improvement in the specificity and sensitivity of the polyclonal antibody can be realized.

According to a manufacturing method of vitellogenin involving the embodiment, the induction of vitellogenin synthesis is carried out in a frog body, blood is sampled from the frog followed by sampling a serum, and the serum is fractionated and purified. As a method of isolating and purifying a serum, centrifugal separation, gel-filtration column chromatography and so on can be properly combined, and thereby vitellogenin can be efficiently and accurately manufactured.

### (EXAMPLES)

In what follows, the present invention will be detailed with reference to examples. However, it goes without saying that the invention is not restricted thereto.

### <Example 1. Vitellogenin assay with Xenopus laevis>

(1) Equipment: protein purification system, micro-plate reader (manufactured by Tosoh Corporation)
(2) Materials: male adult Xenopus laevis 17β-estradiol: CAS No.: 50-28-2, molecular formula, molecular weight: 272.4 Lot No.: 19C-0519 (available from SIGMA)
   anti-vitellogenin rabbit antiserum: one prepared in year of 1979 and preserved at -80 degrees centigrade was used.
(3) Example of manufacture of vitellogenin antigen
   In order to obtain vitellogenin, to a male adult Xenopus laevis, a propylene glycol solution containing 10 mg/ml of 17β-estradiol (hereinafter referred to as E2) was injected at a concentration of 30 µg/g BW (Body Weight) and thereby the induction of vitellogenin synthesis was carried out. Blood sampled after 8 days' breeding was coagulated, followed by centrifuging at 15000 rpm for 5 min at 4 degrees centigrade, and thereby a supernatant (blood serum) was sampled. Every 1 ml of the blood serum sample was isolated and purified by use of an anion exchange chromatography system (QAE-Sephadex, manufactured by Bio-Rad Econosystem) and thereby a vitellogenin preparation was obtained. With BSA (bovine serum albumin) as a reference protein, the vitellogenin preparation was quantified with a BCA reagent followed by rendering a 50 percent glycerol solution at 0.5 mg/ml further followed by preserving at -20 degrees centigrade. This was used as a standard vitellogenin solution in experiments below.
(4) Example of manufacture of vitellogenin antibody
   From 10 ml of anti-vitellogenin rabbit antiserum frozen and preserved at -80 degrees centigrade, according to the ammonium sulfate fractionation, IgG antibodies were recovered. In order to purify antibodies against vitellogenin, the recovered IgG antibody solution was purified by use of a Sepharose 4B adsorption column thereto blood serum proteins of a normal male Xenopus laevis are conjugated. Furthermore, by use of a purified vitellogenin-conjugated Sepharose 4B column, the affinity purification was applied. In order to prepare labeled antibodies, to the purified antibodies, according to a periodic acid oxidation method, horseradish peroxydaze (HRP) was covalently coupled (HRP labeled polyclonal antibody).
   Thus, two kinds of the adsorption-purified polyclonal antibodies and the affinity-purified HRP-labeled polyclonal antibodies were used as antibody solutions in experiments below.
(5) Vitellogenin detection method due to Western blotting (comparative example)
   A gel of SDS-7.5 percent acrylamide was prepared, 0.05 µl of each of blood serum of an adult male Xenopus laevis administered as mentioned above with E2 by injection and normal male blood serum thereof and a dilution sequence in the range of 7.5 to 750 ng of purified vitellogenin antibodies were electrophoresed, and isolated proteins in the gel were blotted on a membrane according to a semi-dry blotting method. An immunoreaction was performed with 1 µg/ml of vitellogenin polyclonal antibodies followed by reacting with HRP labeled anti-rabbit IgG goat antibodies, and vitellogenin were detected on an X-ray film by means of a chemical fluorescence development method. Thereby, the specificity of the antibody and the detection sensitivity due to the western blotting were verified.
(6) Vitellogenin detection method by ELISA
   The vitellogenin detection due to the ELISA was carried out according to two methods of a) sandwich method and b) competitive method, and with a dilution sequence of a purified vitellogenin preparation, the detection sensitivities were compared and studied.
   The respective detection procedures were determined as follows after preliminary experiments.

### Example 1a: competitive method

### 1. Immobilization of vitellogenin antibody

Each of 50 µl of standard vitellogenin diluted at 5 µg/ml with PBS was dispensed on a microplate followed by incubating at 37 degrees centigrade for 2 hr.
1. Plate washing (0.1 percent Tween 20-PBS)
2. Blocking of plate (0.5 percent I-Block, 0.1 percent Tween 20-PBS)
3. Competitive reaction of antigen and HRP-labeled polyclonal antibody
   On a separate plate, 30 µl of antigens and 30 µl of 1 µg/ml polyclonal antibodies (diluted with a blocking solution) labeled with HRP were mixed followed by incubating at room temperature for 1 hr.
4. Washing of solid-phased plate (0.1 percent Tween 20-PBS)
5. Immune reaction to solid-phased antigen
   Fifty micro-litters of a blend solution of 4 are transferred on an solid-phased plate, followed by incubating at room temperature for 1 hr.
6. Plate washing (0.1 percent Tween 2-PBS)
7. Peroxidase reaction with chromogenic substrate ABTS
8. Absorbance measurement at 405 nM with a plate reader

### Example 1b: Sandwich method

1. Immobilization of vitellogenin antibodies
   Each of 50 µl of anti-vitellogenin antibodies diluted at 5 µg/ml with PBS was dispensed on a microplate followed by incubating at 4 degrees centigrade overnight.
2. Plate washing (0.1 percent Tween 20-PBS)
3. Blocking of plate (0.5 percent I-Block, 0.1 percent Tween 20-PBS)
   Reaction of a test body or a standard antigen and an solid-phased antibody: at room temperature for 2 hr, the antigen being diluted with a blocking solution.
4. Plate washing (0.1 percent Tween 20-PBS)
5. Reaction with HRP-labeled vitellogenin polyclonal antibody
   Fifty micro-litters of anti-vitellogenin antibody that is labeled with HRP and diluted at 2 µg/ml with a blocking solution were added followed by incubating at room temperature for 1 hr.
6. Plate washing (0.1 percent Tween 2-PBS)
7. Peroxidase reaction with chromogenic substrate ABTS
8. Absorbance measurement at 405 nM with a plate reader (8) Experimental results

### ○ Specificity of polyclonal antibody

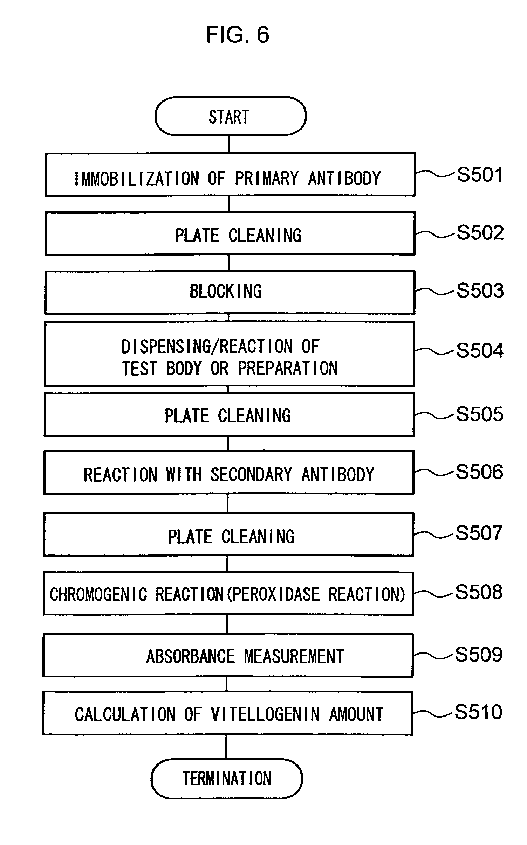

With blood serum of an adult male Xenopus laevis injected with E2 and blood serum of a normal adult male thereof, the western blotting was carried out, followed by applying immunostaining owing to the present antibody. When an antibody before purification was used, a protein common to both blood serum was detected (Fig. 6A). The specificity of the antibody to vitellogenin was heightened owing to one-time adsorption column purification and the immunization reaction with serum protein of a normal adult male was not caused (Fig.6B).

### ○ Detection sensitivity of ELISA

In a vitellogenin detection method that uses the antibody, three methods of the ELISA competitive method, the ELISA sandwich method and the Western blotting method were compared of the detection sensitivity under the conditions set at present time.

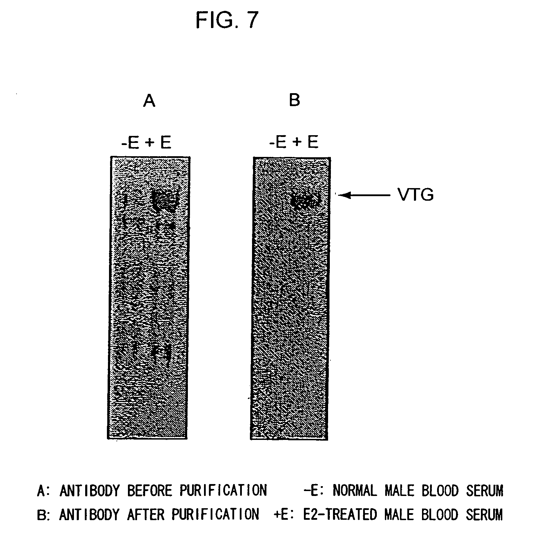

The detection sensitivities were verified of two methods according to the ELISA by setting a standard vitellogenin concentration in the range of 0.1 to 4000 ng/ml. As a result, while the minimum detection limit (3SD) according to the competitive method was substantially 20 ng/ml (Fig. 7A), the minimum detection limit (3SD) according to the sandwich method was substantially 3 ng/ml. Accordingly, it was found that the sandwich method is higher in the sensitivity when the vitellogenin is detected on a lower concentration side. However, a quantification limit that shows the linearity was substantially 30 ng/ml.

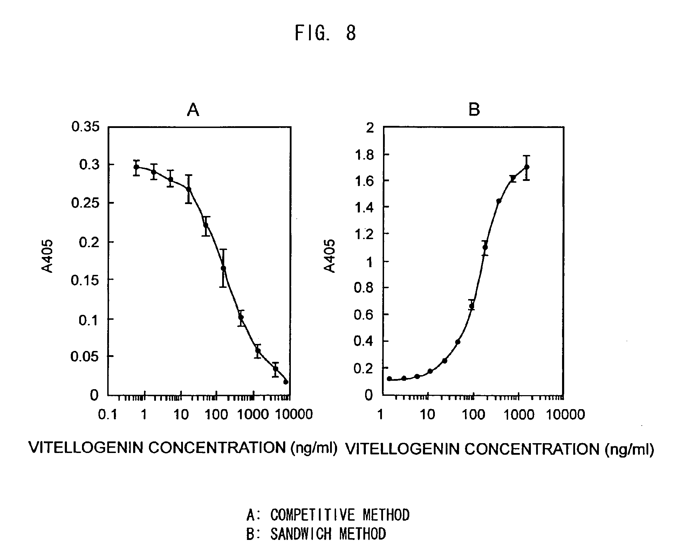

Furthermore, as a comparative example, with an amount of vitellogenins set in the range of 7.5 to 750 ng, the Western blotting method was applied to detect the concentration and substantially 10 ng could be detected (Fig.8).

### (8) Considerations

In order to evaluate influences of the environmental chemicals on the vitellogenin synthesis, it is considered that a measurement method that can detect the vitellogenin synthesized in an adult male at a concentration as low as possible is necessary. Accordingly, studies below were performed.

Frozen and preserved antiserum was adsorbed with a normal male serum protein-conjugated column and thereby the specificity to the vitellogenin was remarkably improved. An improvement in the specificity is considered effective in detecting vitellogenin at lower concentrations.

Difference of the sensitivities of two kinds of the ELISA detection method was compared of the minimum detection limits. While the detection limit of the ELISA-competitive method was substantially 20 ng/ml, the detection limit of the ELISA-sandwich method was substantially 3 ng/ml. Dispersion of measurements on a lower concentration side was large in the competitive method and small in the sandwich method. Accordingly, the sandwich method was more effective in detecting low concentration antigens. Furthermore, it is considered that when antibodies are sufficiently solid-phased and thereby a collision frequency between antigens and antibodies is heightened, the sensitivity can be effectively heightened. Accordingly, it is considered effective to process a plate with a reagent and so on to improve an immobilization efficiency of antibodies.

Furthermore, as a comparative example, the detection sensitivity of the Western blotting was studied. As a result, quantitative detection was possible at substantially 10 ng or more. The Western blotting tends to be lower in the detection sensitivity than the ELISA method, is troublesome in operations and takes a longer time to detect; however, it can advantageously qualitatively confirm antigens. The vitellogenin is likely to be decomposed, and in the western blotting, decomposition products may not be detected. Accordingly, it is considered that by combining both the ELISA and the western blotting method, influences of environmental chemicals on the synthesis of vitellogenin can be correctly evaluated.

### <Example 2. Xenopus laevis vitellogenin assay>

### Example 2-1: Comparison between exposure test methods

In an exposure test, two methods of adding E2 to breeding water and of injecting E2 to individual organisms were carried out and compared.

### 1) Materials and exposure method

### A. Exposure in water

Adult male Xenopus laevis of same age (two years and half old) were used. Every five thereof were bred in each of a water bath where one in which in 20 L of dechlorinated tap water E2 dissolved in DMSO was added was filled (experimental group) and a water bath where one in which in 20L of dechlorinated tap water the same amount of DMSO that is a solvent was added is filled (comparative group). Exposure concentrations of the E2 were five levels of 0.1 nM, 1 nM, 10 nM, 100 nM and 1000 nM, and, at 3.5 days during a breeding period of 7 days, the breeding water was once exchanged. A water temperature was set at 22 degrees centigrade, bright and dark periods each were set at 12 hr, and during a test period bait was not fed.

### B. Injection method

Adult male Xenopus laevis same in the age as A were used. Five thereof in which an E2 solution was injected by means of injection (experimental group) and another five thereof in which propylene glycol that was a solvent was injected by use of an injection by substantially 200 µl (comparative group) were bred in a glass water bath filled with 20 L of dechlorinated tap water. Exposure concentrations of E2 were five levels of 0.002 µg/g BW, 0.02 µg/g BW, 0.2 µg/g BW, 2 µg/g BW and 20 µg/g BW, and, during a breeding period of 7 days, reinjection was once (at 3.5 days) applied. A water temperature was set at 22 degrees centigrade, bright and dark periods each were set at 12 hr, and during a test period bait was not fed.

At the injection, individual organisms were measured of weight and substantially 200 µl of the E2 solution was injected so as to be a set concentration.

### 2) Sampling method of test body

At the detection of the vitellogenin, two samples of a blood serum and a liver homogenate were used and compared to study.

### A. Sampling of blood serum

1. To an abdominal cavity of a frog, 0.5 to 1.0 ml of an anesthetic agent (20 mg/ml aminobenzoic acid ethyl ester) was injected.
2. An operation was performed, and 0.2 ml of blood was sampled from a pulsating heart with a syringe in a tube and placed on ice.
3. A centrifugal process was applied at 15000 rpm, at 4 degrees centigrade for 10 min, followed by sampling a supernatant.
4. Dilution was applied at a factor of 10 with a 20 mM of an EDTA-0.2 percent Tween 20-PBS solution followed by refrigerating and preserving (in the case of long preservation, by freezing and preserving).

The diluted sample was quantified of the vitellogenin according to the ELISA sandwich method.

### B. Sampling of liver homogenate

1. After the sampling of blood, a liver piece was cut to substantially 0.05 to 0.1 g followed by weighing.
2. In a tube where the liver piece put, 1 ml of 20 mM EDTA-0.5 % Tween 20-PBS solution was added.
3. The liver was homogenized over ice.
4. A centrifugal process was applied at 15000 rpm, at 4 degrees centigrade for 10 min, followed by sampling a supernatant, further followed by refrigerating and preserving (in the case of long preservation, by freezing and preserving).

The diluted sample was quantified of the vitellogenin by ELISA sandwich.

### 3) Results and considerations

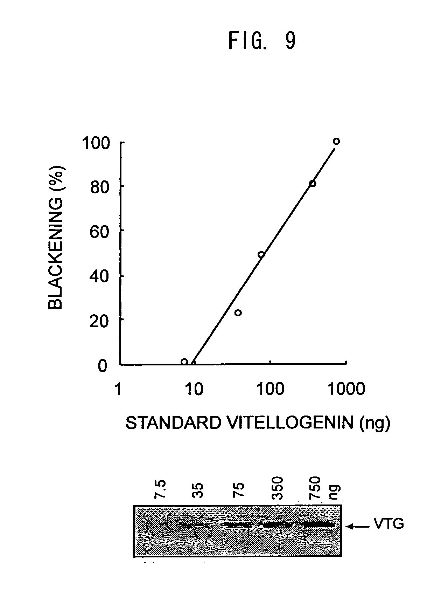

In the exposure in water, response was observed from 10 nM and obviously rose high at 100 nM (Fig. 9). That the synthesis does not increase at 1000 nM coincides with the finding in a cultivated hepatocyte that the synthesis reaches the maximum at a concentration lower than 1000 nM. In the case of the exposure in water, the exposure period was set at 7 days this time; however, the exposure period has to be studied more. Furthermore, this time, the concentrations were varied at a factor of 10 times to test; however, in order to detect the concentration dependent response around 1 nM, the concentrations have to be set at a finer factor and the exposure tests with a lot of individual organisms are considered necessary.

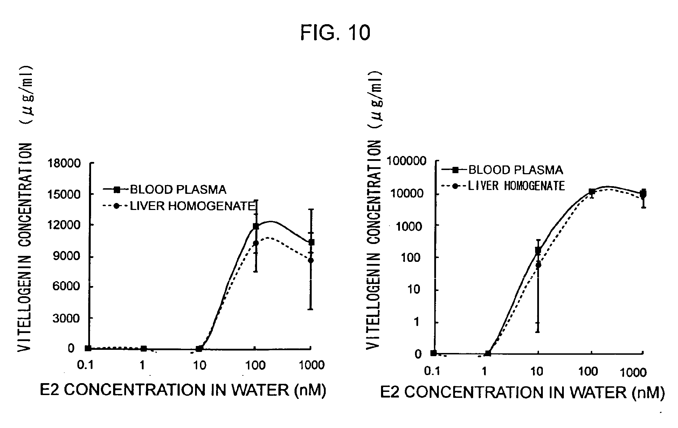

Furthermore, in the injection method, response was detected from around 0.02 µg per body weight and increased dependent on an administered amount (Fig. 10). The exposure in water and the injection cannot be directly compared; however, in the injection method, the administration quantity dependency was observed in a wider range. On the other hand, in the exposure method in water, an abrupt rise was found in the range of 10 to 100 nM. It is not understood how the E2 administered according to the injection method diffuses within an individual organism; however, when it is assumed that uniform diffusion in an individual organism occurs, an amount applied here corresponds to 7.3 nM to 73 µM in a body. On the other hand, in the exposure method in water, a production quantity of the vitellogenin was substantially 13000 µg/ml at 100 nM and that owing to the injection method was substantially 14000 µg/ml at 73 nM. This shows that the exposure method in water can detect estrogen effect at a much lower concentration. It is considered that in the case of the injection method, only part of the injected E2 works in a body. When the quantified vitellogenin concentrations were plotted logarithmically, the E2 concentration dependency on a lower concentration side could be visually expressed (right graphs of Figs. 9 and 10).

In the study of sampling methods of detection bodies, when the liver homogenates are used, measurement values of vitellogenin tend to appear lower than that when blood plasma are used and the dispersion thereof is larger, but patterns of the response curves are substantially same. Accordingly, it is considered that when an adult allowing sampling blood therefrom is used, the use of blood plasma results more preferably; however, it was shown that when a small individual organism such as a larva is used, a liver homogenate could be effectively used as well.

### Example 2-2: Test with primary hepatocyte culture

### 1) Materials and method

### A: Chemicals

○ 10 × perfusate (0.14% KCl, 5.5% NaCl, 0.44% pyruvate, 1% glucose, 2.38% HEPES, 5% BSA and 0.2% NaHCO₃)
○ Collagenase perfusate (1 × perfusate is controlled to pH 7.2 to 7.5 with phenol red to prepare a solution of 0.1% collagenase. Filtration and sterilization)
○ Culture liquid (50% L-15, 1 µg/ml insulin, 0.5% glucose and antibiotics)

### B: Sampling method of hepatocyte

1. A frog is sterilized in potassium permanganate (5 mg/ml) for 2 hr or more (superficial skin color becomes brown).
2. An anesthetic (20 mg/ml-aminobenzoic acid ethylester) is injected by 0.5 to 1.0 ml to an abdominal cavity.
3. A ventral skin is thoroughly sterilized with a cotton bud wetted with alcohol, followed by operating.
4. A heart is exposed, a syringe is inserted from a cardiac chamber, and a collagenase perfusate is sent in through a hepatic vein by use of a perista pump to perfuse a liver.
5. The liver is taken out and transferred in a beaker, followed by cutting small with a pair of scissors.
6. Transferring into an L-tube together with a small amount of collagenase perfusate.
7. Shaking for 30 to 60 min in an incubator at 24 degrees centigrade.
8. Suspending thoroughly with a pipette, followed by filtering through a nylon mesh.
9. A slight amount of culture liquid is added, followed by centrifuging at 300 rpm for 1 to 2 min.
10. A centrifugal supernatant is sucked and removed, followed by centrifuging a culture liquid once more (this operation is repeated twice).
11. A cell concentration is measured with a blood corpuscle measurement plaque, followed by diluting to 100,000 to 200,000 per 1 ml.
12. Seeding every 100 to 300 µl to a microplate (substantially 20,000 pieces/well is better).
13. Cultivating in an incubator at 24 degrees centigrade.

### C: Test method

The processing is carried out according to a method in which a test substance is added to a culture liquid to culture.

After substantially one day on in culture, a hepatocyte retrieves its function, adheres to a plate followed by extending; accordingly, from substantially second day in culture a culture liquid is exchanged to a culture liquid containing a material to be tested; on the eighth day, a culture liquid is sampled followed by detecting vitellogenin according to the ELISA method. The culture liquid is exchanged once every three days (when too many cells are seeded, frequent exchange is necessary). In the case of the E2 processing, it is known that from the third day on after the processing vitellogenin can be detected in the culture liquid; however, since survived hepatocytes in culture are stable for three weeks, for the test of a substance low in the activity, evaluation is carried out for a longer period.

### 2) Results and considerations

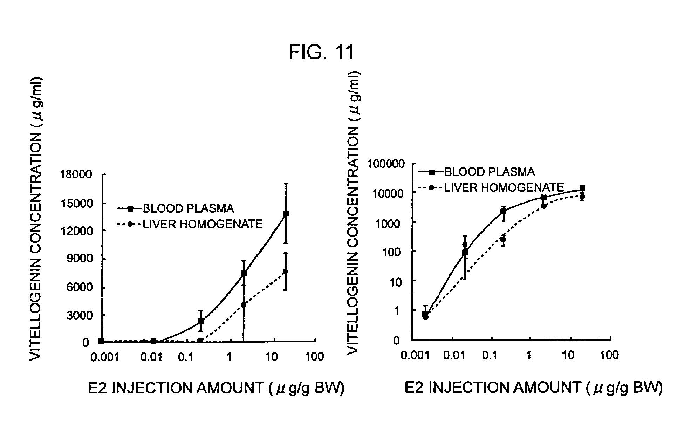

In the case of assay that uses a hepatocyte in culture, at an exposure concentration of 0.6 nM or more of E2, a significant increase in the vitellogenin concentration dependent on the concentration was recognized (Fig. 11). In comparison with the case where individual organisms are used, the dispersion of measurements is small, that is, the reproducibility is higher. In particular, on a lower concentration side than that in the exposure test in water with individual organisms, the vitellogenin synthesis dependent on the E2 concentration was recognized.

As other advantages in the use of the hepatocytes in culture,
· being economical because many samples can be tested simultaneously,
· being speedy and convenient because owing to the use of secreted protein a culture liquid can be used as that in the test,
· being capable of directly detecting response of the hepatocyte without being adversely affected owing to hormones in living organisms,
· being capable of setting strict exposure conditions, and
· making a special technology unnecessary because a detection system can be compiled in a manual can be cited.

### <Example 3. Making vitellogenin assay more sensitive>

In example 3, in order to evaluate influences of chemicals having a estrogenic hormone action on amphibians, the vitellogenin (VTG) assay that uses a male Xenopus laevis was tried to make more sensitive. For this, polyclonal antibody against Xenopus laevis VTG was prepared anew, an ELISA kit for Xenopus laevis VTG was optimized and the ELISA kit was prepared, followed by evaluating the measurement accuracy.

### A. Method

### 1. Preparation of polyclonal antibody against Xenopus laevis VTG

### (1) Purification of Xenopus laevis VTG polyclonal antibody

To an adult male Xenopus laevis, a propylene glycol solution containing 10 mg/ml of 17β-estradiol (E2) was injected at an amount corresponding to 30 µg/g body weight, thereby induction of VTG synthesis was carried out. Every 0.5 ml of blood serum sampled after breeding for 10 days was isolated and purified by use of an anion exchange chromatography system (trade mark: QAE-Sephadex, manufactured by Bio-Rad Econosystem).

### Separation conditions

Column: QAE-Sephadex A50 was swollen with an A liquid (below) and packed in an open column having a diameter of 1 cm and a length of 10 cm.
Flow rate: 0.5 ml/min
Fraction: 3 ml/test tube
Buffer gradient conditions
· A liquid: 0.1 M Tris-HCl (pH 6.5)
· B liquid: 0.1 M Tris-HCl (pH 6.5)/0.5 M NaCl

| Time (min) | % B Liquid |
|---|---|
| 0 | 0 |
| 20 | 0 |
| 200 | 0 - 80 |
| 260- | 80 |

In order to concentrate the VTG solution, a gel filtration carrier (Sephadex-G25) was used. In the quantification of the concentration, a BCA (Bicinchoninic Acid) Protein Assay Kit (PIERCE) was used and calculated in terms of BSA (bovine serum albumin). In order to confirm the purity, SDS-7.5% acrylamide gel electrophoresis/CBB (Coomassie brilliant blue) staining was carried out. The VTG-purified product was used to prepare immunizing antigen and an ELISA standard, and the rest was preserved as a 50% glycerol solution of 0.5 mg/ml at -20 degrees centigrade.

### (2) Immunization, and purification and labeling of antibody

In order to prepare polyclonal antibody, immunization was applied to a rabbit. From a rabbit whose antibody titer value has risen, in turn, exanguination was carried out to prepare IgG fractions. In order to purify antibody against the VTG of Xenopus laevis, an adsorption purification column and an affinity purification column were prepared.

### Preparation of adsorption purification column

Two milliliter of serum blood of a normal male Xenopus laevis (containing substantially 100 mg of protein) and 5 g of CNBr-activated Sepharose 4B were covalently coupled owing to a coupling reaction.

### Preparation of affinity purification column

Fifty milligrams of Anti-VTG antibody of a Xenopus laevis and 7.5 g of CNBr-activated Sepharose 4B were covalently coupled owing to a coupling reaction.

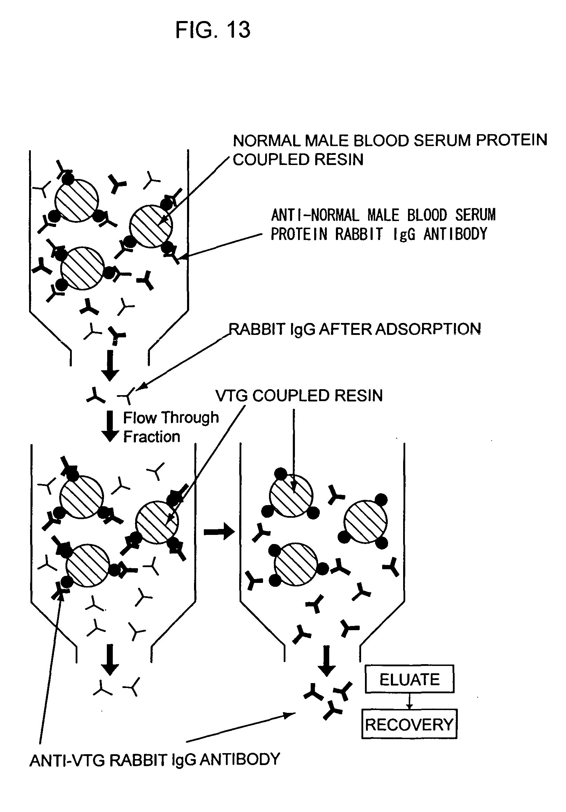

As shown in Fig. 13, the IgG fraction of immunized rabbit blood serum (substantially 200 mg) was mixed with the adsorption purification column, followed by shaking at room temperature for 30 min. This was filtered and thereby antibodies to proteins in the blood serum of a normal male Xenopus laevis were eliminated. Furthermore, the filtrate was mixed with the affinity purification column and shaken at 4 degrees centigrade overnight, followed by eluting antibodies bonded to the column. A rise in the specific antibody titer due to purification was confirmed by means of the ELISA method.

Still furthermore, the obtained affinity-purified polyclonal antibodies were partially covalently bonded with horseradish peroxidase (HRP) according to a periodate oxidation method (Conjugation of Horseradish Peroxidase to Antibodies: Current Protocols in Molecular Biology, 11.1.2), and thereby HRP-labeled polyclonal antibodies were prepared.

### (3) Confirmation of the specificity of antibody

A SDS-7.5% acrylamide gel was prepared, 0.025 µl equivalent of each of blood serum of an adult male Xenopus laevis injected with E2 (substantially 1 mg) and blood serum of a normal male Xenopus laevis and 100 ng equivalent of purified VTG antigens were electrophoresed, and proteins separated in the gel were blotted on a membrane according to a semi-dry blotting method. An immunozation reaction was applied at anti-VTG affinity-purified polyclonal antibodies 1 µg/ml, followed by reacting with HRP-labeled anti-rabbit IgG goat antibodies (secondary antibodies), and the VTG was detected on an X-ray film according to a chemical fluorescence staining method. Thereby, the specificity of the antibodies was verified.

### 2. Optimization of ELISA KIT

As mentioned above, since it was found that the ELISA sandwich method is more sensitive than the ELISA competitive method in a lower concentration region, an ELISA KIT based on the sandwich method was prepared. The sandwich method will be roughly shown below. In addition, of newly prepared VTG polyclonal antibodies of a Xenopus laevis, the ELISA method was tried to optimize at the respective reaction steps.

### Outline of ELISA sandwich method

1. Immobilization of antibody on microplate (adsorption-purified Anti-VTG antibody 50 µl, under a condition of 4 degrees centigrade, and overnight)
   Washing: cleaning liquid 300 µl, 3 times, and 30 min
2. Blocking of a plate surface with a blocking reagent
3. Reaction in sample or standard liquid 50 µl, at room temperature, for 1 hr
   Washing: cleaning liquid 300 µl, and 3 times
4. Reaction of labeling antibody (HRP-labeled anti-VTG antibody 50 µl, room temperature, and 1 hr)
   Washing: cleaning liquid 300 µl, and 3 times
5. Staining (staining liquid 100 µl, room temperature, and standing for 30 min to 1 hr)
6. Measurement of the absorbance at 405 nm with a plate reader

### (1) Optimization of ELISA reaction conditions

### i) Concentration of solid-phased VTG antibodies

A concentration of the affinity-purified VTG polyclonal antibody that is immobilized on a plate was set at 1.25, 2.5 and 5 µg/ml, the immobilization was carried out at room temperature for 1 hr, thereby in the range of 0 to 1000 ng/ml of the standard VTG a calibration curve was prepared. Furthermore, with one that was solid-phased at an appropriated concentration of 1.4 µg/ml at 4 degrees centigrade overnight, an experiment to get calibration curve was simultaneously carried out. By comparing results, an optimum concentration to get solid-phased VTG antibodies was studied.

### ii) Concentration of HRP-labeled anti-VTG antibody

It is considered that the detection sensitivity according to the ELISA method depends on a labeling efficiency of HRP to the VTG polyclonal antibody. Accordingly, a concentration of the HRP-labeled Anti-VTG antibody used has to be optimized. Of the HRP-labeled Anti-VTG antibody prepared this time, a concentration was set at 1, 2 and 4 µg/ml and an optimum concentration of the HRP-labeled Anti-VTG antibody in the ELISA method was studied.

### iii) Composition of diluting solution of blood plasma sample-addition recovery test-

In order to study conditions involving when the VTG in blood plasma sample of a Xenopus laevis is measured, addition recovery tests were conducted to improve the ELISA method. Of a normal male blood plasma, a dilution sequence (1/20, 1/200, 1/1000, 1/5000, 1/20000 and 1/100000) was prepared, and the standard VTG was added to each sample thereof so as to be 500, 100, and 20 ng/ml to prepare samples. As a dilution solution for use in diluting the blood plasma, a sample dilution solution (0.5% Snow Brand Block Ace-0.1% Tween 20-10mM EDTA-PBS) and one in which BSA is added in a sample dilution solution so as to be finally 1% were used. Based on calibration curves with the respective dilution solutions, VTG concentrations in the samples were quantitatively determined and thereby recovery rates to added amounts were calculated. Furthermore, in order to confirm the dilution-dependent linear regression, diluted with a dilution solution to which 1% BSA was added, VTG in blood plasma samples 1 and 2 (both are male blood plasma exposed in water to 10 nM E2 for 7 days) were measured in a dilution sequence from 1/400 to 1/2 dilution, thereby VTG quantification was carried out.

### (2) Optimization of method of blood plasma sample preparation

### i) Blood sampling method from living organisms (study of where blood is sampled)

As a blood sampling method from a living organism of a Xenopus laevis, the bleeding from three positions of a body flank: subdermic blood vessels gather, a paddle where blood vessels can be easily seen and a nail removed surface were carried out to optimize in quantity of the blood.

### 3. Evaluation of ELISA KIT

(1) Preparation of calibration curve
   A calibration curve was prepared by use of the ELISA KIT. From a VTG solution adjusted to 1000 ng/ml, 1/3 or 1/2 dilution series (1000 ng/ml to 2 ng/ml, and 0 ng/ml) were prepared, and a kit protocol was followed.
(2) Evaluation on accuracy of the detection system
   With a Xenopus laevis VTG standard solution, a calibration curve was prepared following a quadruple measurement, a 95% reliable by minimum detection limit value (an average value at a point of zero VTG concentration + 2 x standard deviation (2SD)) was obtained, and the sensitivity was evaluated. Furthermore, the relative standard deviation at each of measurement points (%CV) was obtained, and the accuracy thereof was evaluated.
4. Others (of new findings and an application method of the ELISA KIT)

### (1) New finding about labeling method

As a finding leading to further higher sensitivity of the ELISA KIT, results were obtained of the ELISA that uses biotinylated antibody and HRP labeled streptavidin.

### (2) Interspecies cross-reactivity of antibody

In order to investigate the cross-reactivity of the antibody against VTGs of frogs other than the Xenopus laevis and an extent thereof, E2 (substantially 1 mg) was injected to a Xenopus tropicalis that is a closely-related species of the Xenopus genus, a Bombina bombina of the Bombina genus and a Rana rugosa, a Rana limnocharis Boie and a Rana nigromaculata of the Rana genus, and a Japanese tree frog of the Hyla genus, and a Schelegel's green tree frog of the Rhacophorus genus, followed by breeding for 6 days, further followed by sampling blood serum. Each of the blood serum was diluted stepwise from 1/200 to quantify the VTG according to the ELISA, from the VTG calibration curve of the Xenopus laevis a VTG concentration was estimated, and thereby an immuno reactive amount of VTG of a frog of other genus was obtained. In the next place, based on the immuno reactive amount, each of frog blood serum proteins was separated with SDS-PAGE so that an amount of the VTG that is recognized by the antibody may be equivalent, followed by CBB staining. By comparing a density of a CBB stained band at a position of substantially 200 kDa in a CBB stained gel photograph, a degree of the cross-reactivity was expressed.

### (3) Xenopus laevis hepatocyte in primary culture

As one of applications of the ELISA KIT, an application to the quantification of VTG concentration in a liquid Xenopus laevis hepatocyte culture was studied as well. According to a method same as that used in the abovementioned example, hepatocytes of an adult male Xenopus laevis were primarily cultured, followed by culturing in a culture liquid containing a known estrogenic substance such as E2, Estrone (E1) or Estriol (E3). Of a culture liquid after 6 days from the start of culturing (exchanged on the third day), the quantification of the VTG concentration was carried out with the ELISA KIT.

### B. Results

### 1. Preparation of VTG polyclonal antibody of Xenopus laevis

### (1) Purification of VTG antigen

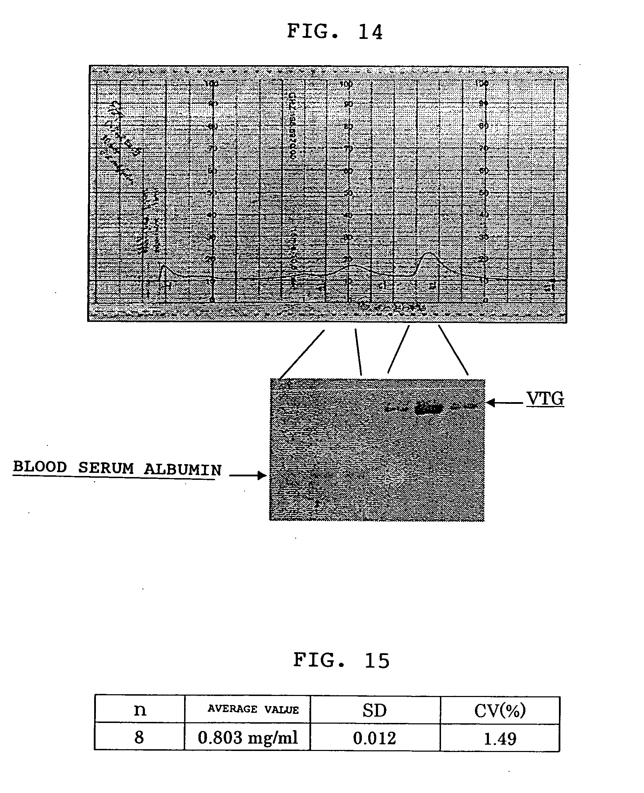

By use of an anion exchange chromatography system, every 0.5 ml of blood serum of an adult male Xenopus laevis in which VTG synthesis was inducted owing to the injection of E2 was isolated and purified. As shown in Fig. 14, from after substantially 140 min on, peaks containing VTG appear on a UV recorder. The last peak is due to the VTG, and one before that is due to blood serum albumin.

Substantially 10 fractions including a peak due to the VTG were confirmed by with SDS-PAGE/CBB staining method (Fig. 14) and fractions that do not contain the serum albumin were collected.

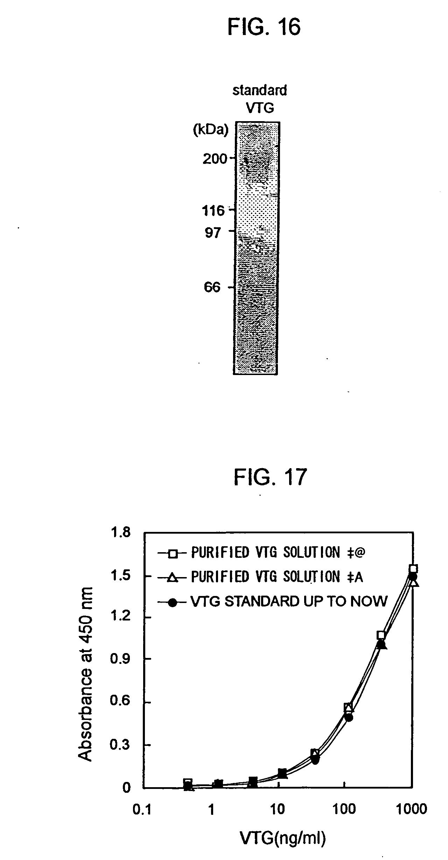

With a BCA reagent, in using of the BSA as standard protein, a concentration of the VTG solution was determined. As a result of 8-fold multiple measurement, the concentration variation was less than 1.5% (Fig. 15). The SDS-PAGE/CBB staining resulted in detecting at a position of a molecular weight of substantially 200 kDa (Fig. 16). On the basis of the determined concentrations of a new and old VTG standards, were quantitated according to the ELISA method, the calibration curves coincided each other (Fig. 17). Abovementioned appropriateness was carried out for each of purification lots, and thereby VTG standards can be stably supplied. The VTG standard other than one that was used for preparation of the ELISA KIT was rendered a 50% glycerol solution and preserved at -20 degrees centigrade. It was found that, thereby, the VTG could be inhibited from denaturing and precipitating; accordingly, it could be preserved for a long period. Finally, from blood serum of an adult male Xenopus laevis injected with E2 by an amount corresponding to 30 µg/g body weight, substantially 13 mg of the VTG was purified.

### (2) Purification and labeling of antibody

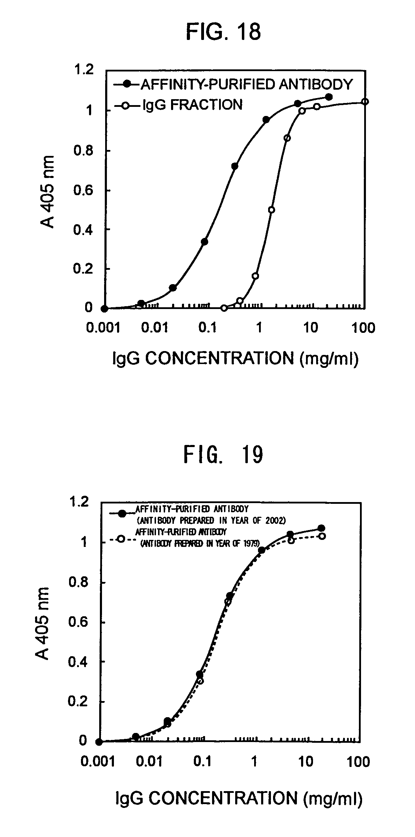

An IgG fraction obtained from a rabbit immunized with VTG antigen was subjected to the adsorption purification process and the affinity purification process. As a result, of the Xenopus laevis VTG polyclonal antibody, a rise in the specific antibody titer of substantially 10-fold was found (Fig. 18). Furthermore, when the specific antibody titer was compared with that of one obtained by subjecting Xenopus laevis VTG polyclonal antibody prepared in 1979 (one preserved at -80 degrees centigrade in a state of antiserum before IgG purification) to the adsorption purification process and the affinity purification process, these specific antibody titers were substantially identical (Fig. 19). It shows that even a polyclonal antibody obtained owing to different immunization, when subjected to the affinity purification process, can obtain an antibody that exhibits the immunozation reactivity same in extent.

### (3) Confirmation of the specificity of antibody against VTG

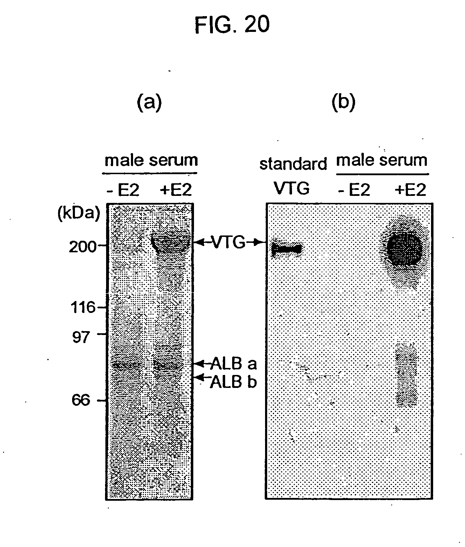

From each of blood serum of a Xenopus laevis adult male injected with E2 and blood serum of a normal adult male, 0.025 µl equivalent was isolated with SDS-PAGE (Fig. 20A), followed by immunostaining owing to a newly prepared antibody. Owing to the affinity purification, the specificity of the antibody to VTG can be secured, and immunoreaction with normal adult male serum protein was not exhibited (Fig. 20B). Thereby, it was confirmed that a polyclonal antibody highly in the specificity to the VTG was obtained. Furthermore, the VTG in the Xenopus laevis blood serum is likely to be decomposed and decomposition products were slightly found as lower molecular weight side.

### 2. Optimization of ELISA KIT

### (1) Optimization of conditions of ELISA reaction

### i) Concentration on solid-phased Anti-VTG antibody

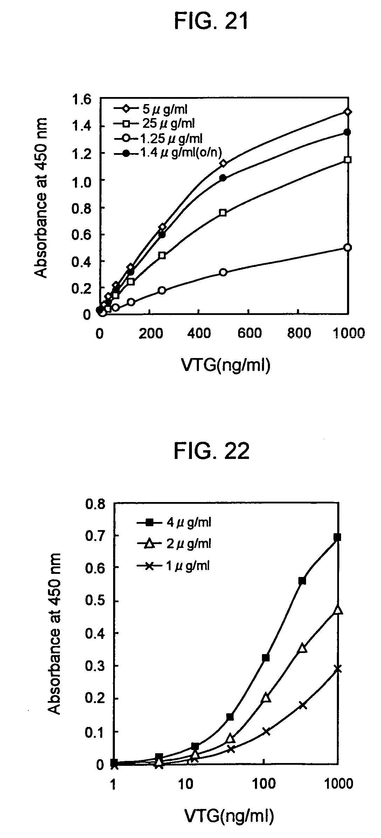

The affinity-purified Anti-VTG antibody that is used for immobilization on a plate was set to immobilize at concentrations of 1.25, 2.5 and 5 µg/ml for 1 hr and at a concentration of 1.4 µg/ml overnight, and thereby calibration curves were prepared (Fig. 21). As a result, at 2.6 µm/ml or more, a sufficient reaction was obtained; however, even at a concentration of 1.4 µg/ml, by immobilizing overnight, the reactivity close to results under the immobilization conditions at 5 µg/ml could be obtained. As a result of such a study, the immobilization conditions at the ELISA were set at 50 µl/well of affinity purified Anti-VTG antibody, at a concentration of 1 µg/ml, at 4 degrees centigrade overnight.

### ii) Concentration of HRP-labeled Anti-VTG antibody

Of the HRP-labeled Anti-VTG antibody prepared this time, at concentration conditions of 1, 2 and 4 µg/ml, the ELISA was performed, and thereby calibration curves were prepared (Fig. 22). As a result, as shown in the drawing, as the concentration of the HRP-labeled antibody became higher, the stained became stronger as a whole; however, in view of an increase in a nonspecific reaction of a negative reference when the antibody concentration is too high and an amount of antibody that can be prepared from the same lot, in the lot, the antibody was judged optimally used at a concentration of 2 µg/ml.

### iii) Composition of blood plasma sample dilution solution-addition/recovery test:

Blood plasma of a Xenopus laevis normal male was diluted in series to 1/20 to 1/100000, to each thereof standard vitellogenin was added so as to be 500, 100 and 20 ng/ml, and thereby samples were prepared and quantified.

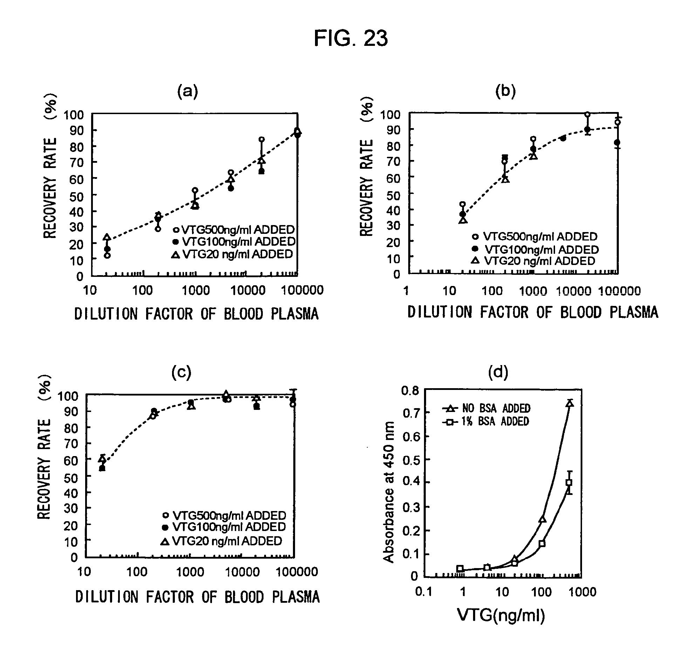

The blood plasma exhibited inhibiting influences, and at 1/20 dilution substantially 85 percent was inhibited. As the blood plasma was diluted, the recovery rate became higher, and at a dilution factor of 100000 or more, a quantification value of the vitellogenin close to an expected value was obtained (Fig. 23A). Furthermore, irrespective of difference of amounts of the vitellogenin added, the recovery rate was substantially constant for each of the blood plasma dilution factors, and error was substantially ± 10 percent. Vitellogenin in the normal male blood serum was below the detection limit of the ELISA method. Subsequently, when BSA was added at concentrations of 0.5 and 1 percent and the addition and recovery test of the VTG was carried out according to a method similar to the above, the higher the BSA concentration was, the lighter the blood plasma inhibiting effect in the recovery rate became (Figs. 23A through 23C). In the case of the BSA being added so that the final concentration in a measurement sample might be 1 percent, when the blood plasma was diluted at least to 1/200, the recovery rate became 90 percent or more (Fig. 23C). Such an improvement in the recovery rate is due to a fact that the reaction inhibition was beforehand included in the calibration curve (Fig. 23D).

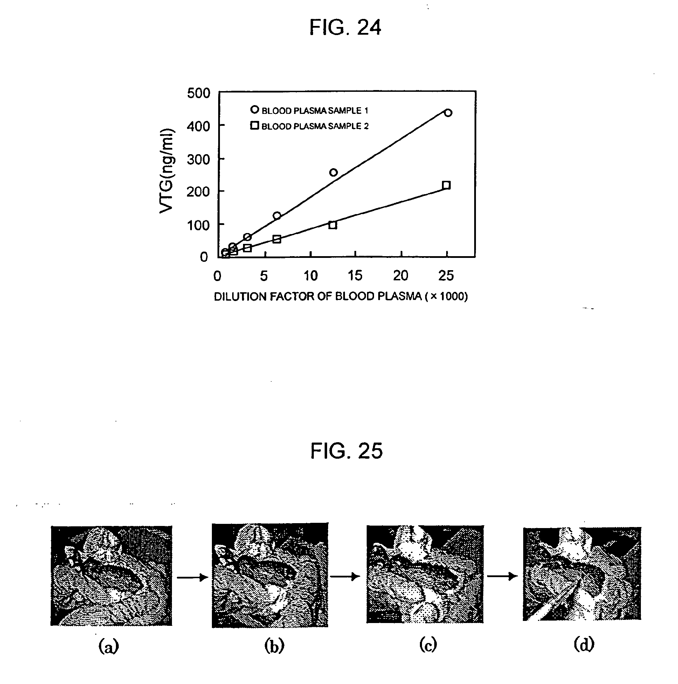

When blood plasma of an adult male in which the VTG induction was carried out owing to actual E2 exposure was diluted and the VTG quantification was carried out, at a dilution factor of 400 or less, the dilution-dependent linear regression of the VTG concentration could be obtained (Fig. 24).

From what was mentioned above, it was determined that, to a dilution solution of a test body, BSA was added so as to be 1 percent in the final concentration.

As a result of the above study, conditions of the ELISA were determined as follows. As to the blocking agent and the chromogenic substrate, alterations were applied.
1) Every 50 µl of an immobilizing affinity-purified Anti-VTG antibody solution (1 µg/ml in PBS) is added to a well, followed by standing at 4 degrees centigrade overnight (Nunc-Immo Plate II),
2) a solution in the well is discarded, with 300 µl of a cleaning liquid (0.1 percent Tween 20-PBS) washing is repeated three times,
3) 300 µl of a blocking solution (0.5% Snow Brand Block Ace-0.1% Tween 20-10 mM EDTA-PBS) is added, followed by standing at room temperature for 1 hr,
4) the solution in the well is discarded, followed by washing once with 300 µl of a cleaning liquid (0.1 percent Tween 20-PBS), and
5) aliquots of 50 µl of solutions obtained by diluting a test body and the standard VTG antigen with a sample dilution solution are added to a well, followed by standing at room temperature for 1 hr.
   · A calibration curve is obtained by preparing a 1/3 dilution sequence (1000 to 1 ng/ml, and 0 ng/ml) from a VTG solution adjusted at 1000 ng/ml VTG.
6) The solution in the well is discarded, followed by washing three times with 300 µl of a cleaning liquid (0.1 percent Tween 20-PBS),
7) the HRP-labeled antibody is diluted with an antibody dilution solution (0.5% Snow Brand Block Ace-0.1% Tween 20-PBS) so as to be 2 µg/ml, 50 µl thereof is added to each of wells, followed by standing at room temperature for 1 hr,
8) the solution in the well is discarded, followed by washing three times with 300 µl of a cleaning liquid (0.1 percent Tween 20-PBS),
9) an aliquot of 100 µl of a staining solution (a TMBZ chromogenic substrate solution is diluted with a chromogenic dilution solution to one hundredth) is added to each of wells, followed by standing at room temperature for 1 hr,
10) an aliquot of 100 µl of a reaction stop solution (1N sulfuric acid) is added to each of wells, and
11) the absorbance at 450 nm is measured with a plate reader (trade name: Multiscan JX manufactured by Dainippon Pharmaceutical Co., Ltd.).

### (2) Optimization of preparation method of blood plasma sample

### i) Blood drawing method from living organism (study on a position where to draw blood)

As a method of drawing blood from a living organism of a Xenopus laevis, the blood drawing methods from three positions, that is, an underarm position where subdermic blood vessels gather, a paddle where blood vessels can be easily seen and a nail-removed surface, were compared and studied to optimize. As a result, the blood drawing from the paddle was judged inappropriate for the present purpose because it was found that ① an amount of blood that oozes is slight, ② oozed blood blurs and the blood drawing is difficult, and ③ a severed blood vessel is rather difficult to heal. The blood drawing from the nail-removed surface was also judged inappropriate as the blood drawing method because it was found that ① an amount of blood that oozes is slight and ② the nail-removed surface takes a long time to heal. A method of drawing blood by stinging a needle to a position from an underarm portion to a flank was judged appropriate as the blood drawing method because ① the blood drawing is easy because oozed blood becomes ball-like and ② a sting is easy to heal. Furthermore, as a result of the optimization of the blood drawing method, a method below was cited as an example of the blood drawing method.

### Blood drawing method (Fig. 25)

1) In a micro-tube, 200 µl of a sample dilution solution is poured, followed by leaving ready for use over ice,
2) a frog is wrapped with tissue paper or the like and grasped firmly with a hand,
3) water of the underarm portion of the frog is removed with tissue paper or the like,
4) a boundary line portion between a back and abdominal side of a lateral region is stung with an injector needle,
5) 1 to 10 µl of oozed blood is measured with a micropipette, added to the sample dilution solution prepared ready for use over ice, followed by preserving over ice until centrifugation is applied, and
6) the micro-tube is centrifuged at 4 degrees centigrade and 8000 rpm for 5 min, a supernatant liquid is transferred to another micro-tube, followed by preserving frozen until measurement as a plasma sample.

### 3. Evaluation of ELISA KIT

### (1) Preparation of calibration curve

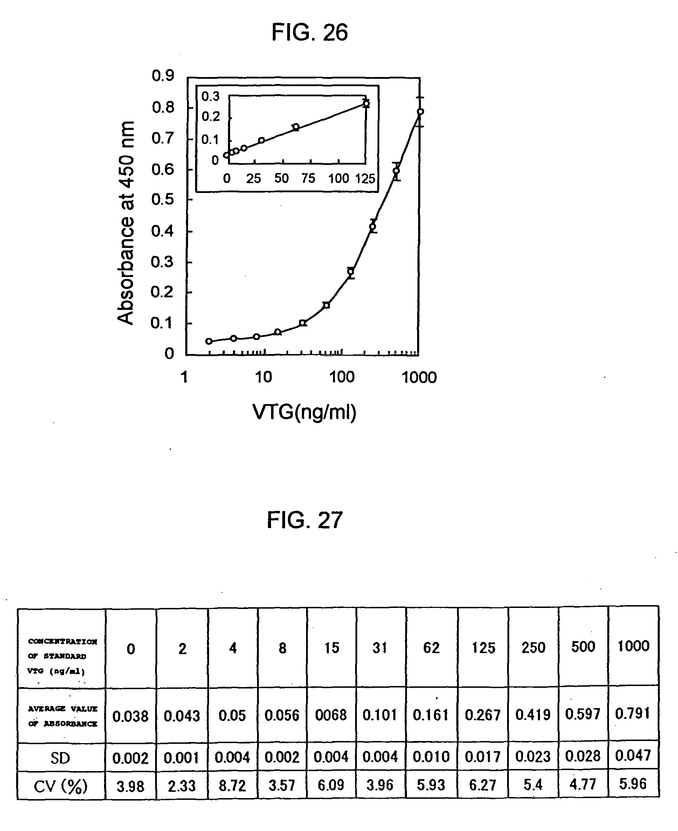

With the ELISA KIT, a calibration curve was prepared. From a VTG solution containing 1000 ng/ml of VTG, a 1/2 dilution sequence (1000 to 2, and 0 ng/ml) was prepared, an ELISA KIT protocol (attached at the end) was followed, and thereby a calibration curve such as shown in Fig. 26 was obtained.

In the obtained calibration curve, the linearity between VTG concentrations 0 and 125 ng/ml was found and the logarithmic linearity was found between concentrations 125 and 1000 ng/ml. Furthermore, though not shown in the drawing, when a double logarithmic chart was prepared, the linearity was found between 8 and 125 ng/ml.

Average values of the absorbance, standard deviations (SD) and relative standard deviations (%CV) at the respective measurement points were as shown in Fig. 27.

### (2) Evaluation of accuracy

From the calibration curve shown in Fig. 26 and numerical values shown in Fig. 27, the accuracy was evaluated.

When a 95% reliable minimum detection limit concentration of the standard VTG at the quadruple measurement was obtained as «an average value at a point where the VTG concentration is 0 + 2SD» , it was found to be substantially 2 ng/ml. Furthermore, when the dispersion of measurements was expressed with a %CV value, an average value of the dispersions was 5% and found to be excellent. Thereby, it was found that the ELISA KIT has the quantitativity in the range of concentrations of 2 to 1000 ng/ml.

### 4. Others (about new finding and application method of the ELISA KIT)

### (1) New finding involving a labeling method

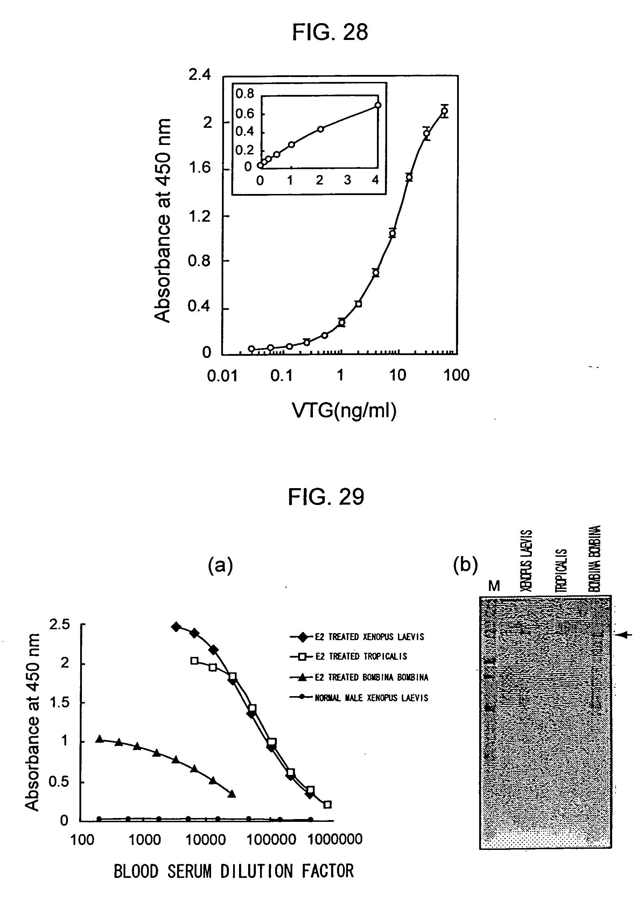

As a finding leading to further higher sensitivity of the ELISA KIT, a new finding was obtained of the ELISA method that uses biotinylated antibody-HRP labeled streptavidin. When a calibration curve was prepared according to the ELISA method that uses biotinylated affinity-purified VTG polyclonal antibody and HRP labeled streptavidin, a calibration curve as shown in Fig. 28 was drawn.

### (2) Interspecies cross-reactivity

By carrying out the ELISA and SDS-PAGE/CBB staining experiment of the interspecies cross-reactivity of antibody, whether the ELISA KIT can be applied as well to the VTG detection of other frogs than the Xenopus laevis or not was studied. E2 (substantially 1 mg) was injected to various kinds of frogs, and after the breeding of 6 days, blood serum were collected. From each of the blood serum diluted to 1/200, a dilution sequence of the serum was prepared, followed by applying the ELISA (Fig. 29A). From a calibration curve that uses a VTG standard of Xenopus laevis, in various kinds of frog blood serum, Xenopus laevis VTG-equivalent values were estimated. In the next place, based on the estimated amount, blood serum of the various kinds of frogs were diluted so that the converted values might be equivalent, followed by SDS-PAGE/CBB staining (Fig. 29B). When density of a band that shows VTG in the CBB staining is identical with that of a band of the Xenopus laevis VTG, it shows that VTG of the frog species is close to 100% in the cross-reactivity with the antibody. From results this time, it was found that only Xenopus tropicalis that is a related species of Xenopus laevis exhibited the cross-reactivity close to 100%. Furthermore, relatively high cross-reactivity was shown also to Bombina bombina VTG. Of other species, the cross-reactivity was low (data are not shown).

### (3) Primary hepatocyte culture

By combining the exposure test to hepatocytes in primary culture and the ELISA, an application to a quantitative screening method of the estrogen activity of various chemicals was studied. B use of a hepatocyte in primary culture of an adult male Xenopus laevis, of known 6 estrogenic agents such as ethynyl estradiol (EE2), diethyl stilbestrol (DES), 17β estradiol (E2), 17α estradiol (α-E2), estriol (E1) and estrone (E3) and chemicals doubted as endocrine disruptors such as bisphenol A (BPA), nonylphenol (NP) and octylphenol (OP), the VTG induction activity was measured.

According to a method similar to (Example 2-2), primary cultured hepatocytes of an adult male Xenopus laevis were prepared, followed by culturing in a culture liquid containing one of the chemicals, and with a culture liquid after 6 days, according to the ELISA, a VTG concentration in the culture liquid was quantified.

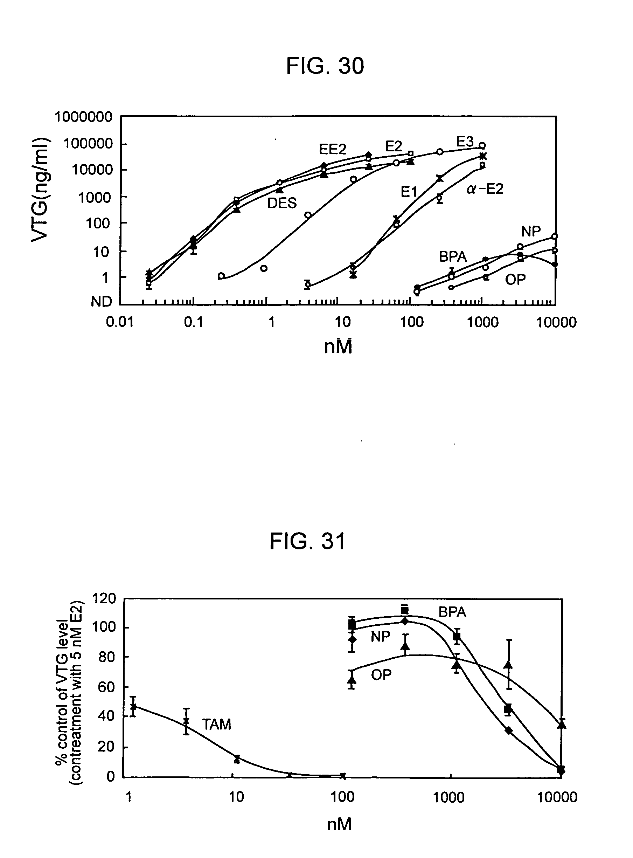

As a result thereof, estrogenic activity dependent VTG synthesis was recognized of the respective chemicals (Fig. 30), in intensities of the estrogenic activity of the respective chemicals, features coincident with the estrogenic activities in the reporter gene assay that uses a known human estrogen receptor were found. Furthermore, the estrogenic activity equivalent to substantially 0.1 nM E2 was detected. Still furthermore, in the case of the quantification being carried out by the ELISA with biotinylated antibody, the estrogenic activity equivalent to substantially 0.03 nM E2 was detected. Thus, the ELISA KIT can be applied also to a test of a primary hepatocyte culture.

Furthermore, by use of the assay system, the antagonist activity of a target substance could be evaluated. For instance, when the hepatocyte was cultured in a culture liquid to which 5 nM E2 and BPA, NP or OP were added, to the VTG synthesis induction due to 5 nM E2 alone, as a concentration of added BPA, NP or OP becomes higher, the antagonistic activity was exhibited (Fig. 31). From the results, it is indicated that the vitellogenin assay that uses a hepatocyte culture can evaluate, of the various chemicals, not only the estrogenic activity but also the anti-estrogenic activity.

### C. Summaries and considerations

### ○ A Xenopus laevis VTG polyclonal antibody was newly prepared and an ELISA KIT was devised. A quantification range of the VTG concentration in the present ELISA KIT was 2 to 1000 ng/ml.

With the newly prepared polyclonal antibody, conditions of a sandwich ELISA method were set and a calibration curve was obtained. While, last year, the 95% reliable minimum detection limit «n = 4, an average value at a point where the VTG concentration is 0 + 2SD» was substantially 3 ng/ml and the quantitativity was maintained up to substantially 300 ng/ml, in the case of the new antibody being used, the 95% reliable minimum detection limit was substantially 2 ng/ml and the quantitativity was maintained up to substantially 1000 ng/ml; that is, the sensitivity was improved. An expansion in the quantification range is assumed due to a change of an antibody solid-phased on a plate from an adsorption purification antibody to an affinity purification antibody. Furthermore, an improvement in the sensitivity on a lower concentration side is assumed due to a change of a chromogenic reagent from an ABTS substrate to a TMBS substrate. When compared with the ELISA method due to medaka monoclonal antibody (2 to 100 ng/ml), the detection sensitivity was substantially same and the quantifiable range was expanded. Furthermore, from a new finding, in a method where a biotinylated antibody and labeled-streptavidin are used, a substantially 30 times increase in the ELISA sensitivity was expected. In future, when a further increase in the sensitivity of the ELISA KIT becomes necessary, a biotinylated antibody of the antibody or a monoclonal antibody specific and high in the affinity has to be prepared.

### ○ The inhibition effect due to blood plasma was balanced by adding BSA to a sample dilution liquid. Furthermore, the recovery rate of the VTG, by diluting the blood plasma at a factor of 200 or more, became substantially 100%. In view of the blood plasma inhibition effect, the minimum detection limit of the VTG concentration in the blood plasma according to the ELISA KIT is approximately 400 ng/ml.

The blood plasma exhibited rather high reaction inhibition effect and at a dilution factor of 20 substantially 85% inhibition was found. As the blood plasma was diluted, the recovery rate became higher, and, at a dilution factor of 100000 or more, quantitatively determined vitellogenin values became values close to expected values. When a blood plasma sample is actually quantified, calibration based on the recovery rates is necessary to be applied; however, it cannot be denied that a procedure becomes troublesome. In this connection, in order to balance the inhibition effect of the blood plasma, a composition of a sample dilution solution was studied. It is considered that owing to a protein contained in the blood plasma, a reaction between VTG and anti-VTG antibody is inhibited for some reasons. Since serum albumin is much contained in the blood plasma, when BSA was beforehand added to a sample dilution solution, the recovery rate of VTG became substantially 100% when the blood plasma was diluted at a factor of 200 or more.

Since the minimum detection limit in the standard VTG is 2 ng/ml, when the blood plasma is diluted at a factor of at least 200, the minimum detection limit of the VTG concentration in blood plasma becomes 400 ng/ml. However, as shown in Fig. 9C, since the recovery rate is substantially 60% at a dilution factor of 20 of the blood plasma, when a VTG concentration in the blood plasma is supposed to be low, a sample preparation is set at 1/20 dilution of the blood plasma, a quantitatively determined value can be calibrated at the recovery rate of 60%. In this case, the minimum detection limit of the VTG concentration in blood plasma becomes substantially 70 ng/ml.

### ○ The present ELISA KIT, when an adult male Xenopus laevis was exposed to E2 of 1 nM or more in water for 7 days, can detect blood plasma VTG.

From the above considerations, when results of exposure tests in water to E2 of an adult male Xenopus laevis in the first example are considered together, it can be said that in the ELISA KIT, the VTG synthesis induction when an adult male Xenopus laevis is exposed to E2 of 1 nM or more in water for 7 days can be detected. It is considered that for the future, with the KIT, the optimization of the test protocol such as change of an amount of VTG induction owing to an exposure duration is carried out, and, at the same time, as needs arise, the ELISA KIT has to be made more sensitive.

### ○ The antibody that was used in the ELISA KIT was confirmed to exhibit the cross-reactivity also to VTGs of frog species (Xenopus tropicalis, Bombina bombina, Rana rugosa, Rana limnocharis limnocharis, Japanese tree frog, Schlegel□s green tree frog, and Black-spotted pond frog) other than Xenopuas laevis.

It was confirmed that the cross-reactivity was high in Xenopus tropicalis and Bombina bombina. Of these two species, it is considered that, by purifying the respective VTGs, the quantification can be carried out with the ELISA KIT. In order to confirm the applicability of the ELISA KIT to other species, it is necessary to study the cross-reactivity in more detail. For that purpose, it is considered necessary to purify VTG of a target frog to clarify the range and limit of applications of the ELISA KIT and, at the same time, of frogs low in the cross-reactivity, as needs arise, necessary to prepare an antibody specific to the species. However, at the moment, since the use of biotinylated antibody and labeled-streptavidin can heighten the sensitivity, these may be applied to frogs of other species low in the cross-reactivity.

### ○ In the estrogenic activity test that uses a primary hepatocyte culture of a Xenopus laevis and the ELISA KIT, similarly to the E2 exposure test of Medaka, the estrogenic activity equivalent to substantially 0.1 nM E2 could be detected.

With a primary hepatocyte culture of a Xenopus laevis, a test was carried out of known substances that have the estrogenic activity and induced VTGs were quantified with the ELISA KIT. As a result, it was characteristically found that difference in the intensities of the estrogenic activities of the respective substances coincides to some extent with that of the estrogenic activities in the reporter gene assay that uses a known human estrogen receptor. Furthermore, the estrogenic activity equivalent to substantially 0.1 nM E2 could be detected. Thus, the ELISA KIT can be applied to a test of a primary hepatocyte culture. According to a new finding, when the ELISA that uses biotinylated antibody was used to quantify, in a test of a primary hepatocyte culture, the estrogenic activity equivalent to substantially 0.03 nM was detected; that is, results identical with the E2 exposure test to Medaka were obtained.

### <Example 4. Manufacture of frog vitellogenin ELISA KIT>

### (1) Manufacture of kit raw materials

### (1)-1 Anti-frog vitellogenin antibody

An example of manufacture according to example 1

### (4) vitellogenin antibody was followed.

### (1)-2 Vitellogenin standard

An example of manufacture according to example 1

### (3) vitellogenin antigen was followed.

### (1)-3 Enzyme-labeled antibody

Peroxidase (Trade name POD, manufactured by Boeringer-ingelheim Co., for use in EIA, Code No. 814393) was dissolved at a concentration of 20 mg/ml in 0.5 ml of a 0.1 M carbonate buffer at pH9.2, followed by blending 0.5 ml of a NaIO4 solution and 0.5 ml of a HRP solution, further followed by reacting at room temperature in a dark place for 2 hr. Thereto, 1.0 ml of an antibody solution (3 mg/ml in 0.1 M phosphate buffer, pH 6.8) was added, followed by keeping for 3 hr in a dark place at room temperature. Thereto, 61 µl of a NaBH₄ solution (5 mg/ml in 0.1 mM NaOH) was added, followed by keeping for 30 min in a dark place at room temperature, further followed by adding 179 µl of a NaBH₄ solution and standing in a dark place for 60 min. Thereto, 2 ml of saturated ammonium sulfate was added, followed by agitating for 30 min over ice. The solution was centrifuged at 4 degrees centigrade and 15000 rpm for 10 min and precipitate was dissolved in 1 ml of a TEN buffer (50 mM Tris-HCl, 1 mM EDTA and 0.9% NaCl). After it was desalted with a span column equilibrated with the TEN buffer, BSA was dissolved at a concentration of 20 mg/ml. For long preservation purpose, a 50% glycerol solution was prepared and preserved at -20 degrees centigrade.

### (2) Manufacture of "anti-frog vitellogenin antibody-solid-phased microplate"

An anti-vitellogenin antibody dissolved in Dulbecco's PBS(-) (Code No. 041-20211, manufactured by Wako Pure Chemical Industries, Ltd) (1 µg/ml) was dispensed on an immobilization plate (trade name EIA/RIA plate strip 8, #2592, manufactured by Costar) so as to be 50 µl/well, followed by standing at 4 degrees centigrade overnight, further followed by washing three times with 300 µl of cleaning liquid (0.05% Tween 20 containing PBS). Thereto, a blocking liquid ((1% Block Ace (trade name: UK-B80, manufactured by Snow Brand Milk Prod. Co., Ltd.) + 1% Sucrose + 10 mM NaCl + 0.05% Slaoff 72N (manufactured by Takeda Chemical Industries, Ltd.) in 5 mM Tris-HCl (pH7.5)) was added so as to be 200 µl/well, followed by standing at 4 degrees centigrade overnight. A total amount was sucked with an aspirator followed by tapping to remove water content. A dewatered and dried immobilization plate was sealed in an aluminum bag, followed by deaerating and sealing with a vacuum dryer, further followed by preserving in a refrigerator at a temperature in the range of 2 to 8 degrees centigrade.

### (3) Manufacture of "powder of vitellogenin standard"

A vitellogenin dilution solution (0.15 M NaCl + 0.01% Slaoff 72N (manufactured by Takeda Chemical Industries, Ltd.) + 4% BSA + 10% sucrose in 50 mM HEPES-Na (pH 7.4)) was diluted so as to be 5 µg/ml, followed by dispensing every 200 µl, further followed by freezing and drying.

### (4) Manufacture of "sample dilution solution (concentration rate of 3)"

In distilled water of 1 L, 6 packs of Dulbecco's PBS(-) (Code No. 041-20211 for use in biochemistry, manufactured by Wako Pure Chemical Industries, Ltd), 1.5 ml of Slaoff 72N (manufactured by Takeda Chemical Industries, Ltd.), 3 ml of Tween 20, 2Na (EDTA · 2Na) (Code No. 343-01861 for use in test and research, manufactured by Dojindo Laboratories), 15 g of Block Ace (trade name: UK-B80, manufactured by Snow Brand Milk Prod. Co., Ltd.) and 30 g of BSA (trade name 7638, manufactured by Sigma) were dissolved, 20 ml each thereof was dispensed in a proper container, followed by preserving at a temperature in the range of 2 to 8 degrees centigrade in a refrigerator.

### (5) Manufacture of "powder of enzyme-labeled antibody"

In an enzyme-labeled antibody dilution solution (0.15 M NaCl + 0.01% Slaoff 72N (manufactured by Takeda Chemical Industries, Ltd.) + 4% BSA + 10% sucrose in 50 mM HEPES-Na (pH7.4)), an enzyme-labeled antibody was diluted so as to be 60 µg/ml, followed by dispensing 100 µl each, further followed by freezing and drying.

### (6) Manufacture of "enzyme-labeled antibody dilution solution"

In distilled water of 1 L, 2 packs of Dulbecco's PBS(-) (for use in biochemistry, Code No. 041-20211, manufactured by Wako Pure Chemical Industries, Ltd), 0.5 ml of Slaoff 72N (manufactured by Takeda Chemical Industries, Ltd.), 1 ml of Tween 20, and 5 g of Block Ace (trade name: UK-B80, manufactured by Snow Brand Milk Prod. Co., Ltd.) were dissolved, after 7 ml each thereof was dispensed in a proper container, a cap was applied thereto, followed by preserving at a temperature in the range of 2 to 8 degrees centigrade in a refrigerator.

### (7) Manufacture of "cleaning liquid (concentration rate of 6)"

In distilled water of 1 L, 12 packs of Dulbecco's PBS(-) (Code No. 041-20211 for use in biochemistry, manufactured by Wako Pure Chemical Industries, Ltd), 3 ml of Slaoff 72N (manufactured by Takeda Chemical Industries, Ltd.) and 6 ml of Tween 20 were dissolved, after 50 ml each thereof was dispensed in a proper container, a cap was applied thereto, followed by preserving at a temperature in the range of 2 to 8 degrees centigrade in a refrigerator.

### (8) Preparation of "chromogenic substrate solution"

Ten milligrams of 5, 5'-tetramethylbenzidine (trade name: TMBZ, Code No. 346-040301 for use in test and research, manufactured by Dojindo Laboratories) was dissolved in 1 ml of dimethylformamide (trade name: DMF, Code No. 045-02916, special grade chemical, manufactured by Wako Pure Chemical Industries, Ltd,), followed by dispensing 250 µl each in a proper brown container and applying a cap thereto, further followed by preserving at a temperature in the range of 2 to 8 degrees centigrade in a refrigerator.

### (9) Preparation of "chromogenic substrate dilution solution"

In 1 L of 40 mM Na₂HPO₄-citric acid buffer solution (pH5.0), 350 mg of urea hydrogen peroxide (trade name: U-1753, manufactured by Sigma) and 0.1 ml of Slaoff 72N (manufactured by Takeda Chemical Industries, Ltd.) were dissolved, followed by dispensing 15 ml each in a proper container and applying a cap thereto, further followed by preserving at a temperature in the range of 2 to 8 degrees centigrade in a refrigerator.

### (10) Preparation of "staining stop solution"

A solution of 1 N phosphoric acid was prepared, followed by dispensing 15 ml each in a proper container and applying a cap thereto, further followed by preserving at room temperature.

By packing thus prepared kit constituents from (1) to (9) and a mixing microplate (167008 manufactured by Nunc) in a box, manufacture of a frog vitellogenin ELISA KIT came to completion.

### <Example 5. Quantification with frog vitellogenin ELISA KIT>

Quantification by use of a frog vitellogenin ELISA KIT prepared according to example 4 is carried out as follows.

### (1) Preparation of sample dilution solution

A sample dilution solution (a concentration factor is three) and distilled water are mixed at a mixing ratio of 1: 2 to prepare a "sample dilution solution".

### (2) Sample preparation

Blood plasma or blood serum is diluted with the "sample dilution solution" prepared according to (1) so as to come into the quantifiable range (3 to 1,000 ng/mL).

### [Preparation method of blood plasma sample]

1) Into an Eppendorf tube, 200 µL of the sample dilution solution is poured, followed by preparing ready for use over ice.
2) A frog is wrapped with a net or tissue paper and grasped firmly with a hand.
3) Water of an underarm portion of the frog is removed with tissue paper or the like.
4) A boundary line portion between a backside and an abdominal side of the underarm is lightly stung with an injector needle (Fig. 25B).
5) One to ten micro-liters of oozed blood (Fig. 25C) is measured and sampled with a micropipette (Fig. 25D), and the sample dilution solution prepared ready for use over ice is added. The tube is preserved over ice until centrifugation is applied.
6) The tube is centrifuged at 4 degrees centigrade and 8000 rpm for 5 min, a supernatant liquid is transferred to another micro-tube, followed by refrigerating and preserving. In the case of the sample being not used for several days, it is preserved frozen up to a time of measurement.

### (3) Preparation of vitellogenin standard solution

The "powder of vitellogenin standard" is dissolved with 200 µL of distilled water (5,000 ng/mL).

Thereafter, by use of the "sample dilution solution", a vitellogenin standard solution having a necessary concentration is prepared. A necessary amount of the 5,000 ng/mL solution is sampled and the rest is refrigerated and preserved.

### [Example of preparation]

By use of an Eppendorf tube or the like, the 5,000 ng/mL solution is diluted to 1/5 with the "sample dilution solution" to 1,000 ng/mL. In the next place, in the microplate, it is diluted in sequence to 1/4 to prepare 1,000, 250, 62.5, 15.6, 3.9 and 0.98 ng/mL. As one for zero concentration, the sample dilution solution is used as that.

### (4) Antigen-antibody reaction -1

To the "anti-frog vitellogenin antibody-solid-phased plate" whose temperature has been returned to room temperature, each of the "sample" and "vitellogenin standard solution" respectively prepared in (2) and (3) is added at 50 µL/well, followed by reacting at room temperature (18 to 25 degrees centigrade) for 60 min.

### (5) Preparation of cleaning liquid

During the antigen-antibody reaction, the "cleaning liquid (concentration factor: 6)" and distilled water are mixed at a blending ratio of 1: 5 to prepare a "cleaning liquid".

### (6) Removal of unreacted matters-1

A reaction liquid is discarded, and 300 µL/well of the cleaning liquid prepared in (5) is used three-times to wash the inside of the well. After the third cleaning liquid is discarded, a microplate that is turned upside-down is lightly tapped with paper towel or the like to completely remove the cleaning liquid.

### (7) Preparation of enzyme-labeled antibody solution

To the "powder of labeled antibody", 3 mL of the 7 mL of the "enzyme-labeled antibody dilution solution" is added and dissolved to prepare a "labeled antibody solution".

### (8) Antigen-antibody reaction-2

A 50 µL aliquot of the "labeled antibody solution" prepared in (7) is added to each well, followed by reacting at room temperature (18 to 25 degrees centigrade) for 60 min.

### (9) Removal of unreacted matters-2

A reaction liquid is discarded, and an aliquot of 300 µL/well of the cleaning liquid prepared in (5) is used three-times to wash the inside of the well. After the third cleaning liquid is discarded, the microplate that is turned upside-down is lightly tapped with paper towel or the like to completely remove the cleaning liquid.

### (10) Preparation chromogenic reagent

The "chromogenic substrate solution" and the "chromogenic dilution solution" are mixed at a mixing ratio of 1: 100 to prepare a "chromogenic reagent".

### (11) Chromogenic reaction/reaction stop

One hundred micro-liters of the "chromogenic reagent" prepared in (10) are added to a well followed by reacting at room temperature for 30 min, further followed by adding 100 µL of the "reaction stop solution" to the well to stop the reaction.

### (12) Colorimetry and calculation of concentration

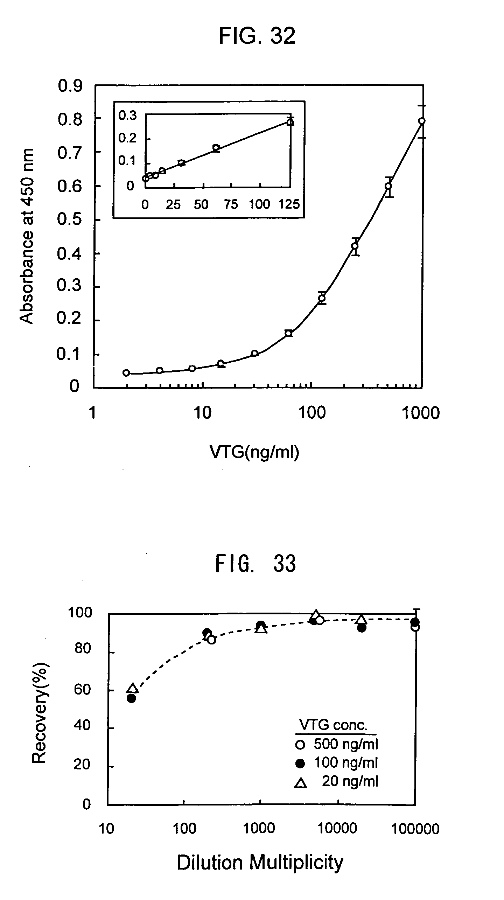

By use of a plate reader, the absorbance at a wavelength 450 nm is measured. A vitellogenin concentration in the sample is calculated from a calibration curve (Fig. 32). Furthermore, by use of a vitellogenin recovery rate curve (the VTG recovery rate versus sample dilution factor) (Fig. 33), measurements are corrected. A quantification range according to the procedure was 3 to 1000 ng/ml. As shown in Fig. 33, it was found that when the blood plasma was diluted at a factor of 200 or more, the recovery rate of 90 to 100% could be obtained.

### <Example 6. Manufacture of high sensitivity ELISA KIT owing to avidin biotinylation>

On the basis of the ELISA KIT according to example 5, a high sensitivity ELISA KIT owing to avidin biotinylation was manufactured.

### (1) Manufacture of raw material kit

### (1)-1 Anti-frog vitellogenin antibody

The example of manufacture according to example 1

### (4) vitellogenin antibody was followed.

### (1)-2 Vitellogenin standard

The example of manufacture according to example 1

### (3) vitellogenin antigen was followed.

### (1)-3 Biotin-labeled antibody

With a Biotin Labeling Kit (Cat. No. 1418 165, manufactured by Roshe), an attached manual was followed.

### (2) Manufacture of "anti-frog vitellogenin antibody-solid-phased microplate"

Example 4 (2) was followed.

### (3) Manufacture of "powder of vitellogenin standard"

Example 4 (3) was followed.

### (4) Manufacture of "sample dilution solution (concentration factor: three)"

Example 4 (4) was followed.

### (5) Manufacture of "powder of biotin-labeled antibody"

In a biotin-labeled antibody dilution solution (0.15 M NaCl + 0.01% Slaoff 72N (manufactured by Takeda Chemical Industries, Ltd.) + 4% BSA + 10% sucrose in 50 mM HEPES-Na (pH7.4)), an enzyme-labeled antibody was diluted so as to be 30 µg/ml, followed by dispensing an aliquot of 100 µl, further followed by freezing and drying.

### (6) Manufacture of "enzyme-labeled streptavidin powder"

In an enzyme-labeled streptavidin dilution solution (0.15 M NaCl + 0.01% Slaoff 72N (manufactured by Takeda Chemical Industries, Ltd.) + 4% BSA + 10% sucrose in 50 mM HEPES-Na (pH7.4)), a HRP-labeled streptavidin (43-8323, manufactured by Zymed) was diluted so as to be 7.5 µg/ml, followed by dispensing an aliquot of 100 µl, further followed by freezing and drying.

### (7) Manufacture of "label dilution solution"

In distilled water of 1 L, 2 packs of Dulbecco's PBS(-) (for use in biochemistry, Code No. 041-20211, manufactured by Wako Pure Chemical Industries, Ltd), 0.5 ml of Slaoff 72N (manufactured by Takeda Chemical Industries, Ltd.), 1 ml of Tween 20, and 5 g of Block Ace (trade name: UK-B80, manufactured by Snow Brand Milk Prod. Co., Ltd.) were dissolved, an aliquot of 15 ml was dispensed in a proper container and a cap was applied thereto, followed by preserving at a temperature in the range of 2 to 8 degrees centigrade in a refrigerator.

### (8) Manufacture of "cleaning liquid (concentration factor: 6)"

Example 4 (7) was followed.

### (9) Preparation of "chromogenic substrate solution"

Example 4 (8) was followed.

### (10) Preparation of "chromogenic substrate dilution solution"

Example 4 (9) was followed.

### (11) Preparation of "staining stop solution"

Example 4 (10) was followed.

By packing thus prepared kit constituents from (1) to (11) and a mixing microplate (167008 manufactured by Nunc) in a box, manufacture of a high sensitivity ELISA KIT due to avidin biotinylation came to completion.

### <Example 7. Quantification with high sensitivity ELISA KIT>

A quantification method by use of a high sensitivity ELISA KIT prepared according to example 6 is carried out as follows.

### (1) Preparation of sample dilution solution

Example 5 (1) is followed.

### (2) Sample preparation

Example 5 (2) is followed.

### (3) Preparation of vitellogenin standard solution

The "powder of vitellogenin standard" is diluted with 200 µL of distilled water (5,000 ng/mL).

Thereafter, with the "sample dilution solution", a vitellogenin standard solution having a necessary concentration is prepared. A necessary amount of the 5,000 ng/mL solution is fractioned and the rest is refrigerated and preserved.

### [Example of preparation]

By use of an Eppendorf tube or the like, the 5,000 ng/mL solution is diluted to 1/80 with the "sample dilution solution" to 62.5 ng/mL. In the next place, in the microplate, it is diluted in sequence to 1/2 to prepare 31.3, 15.6, 7.8, 3.9, 2, 0.98, 0.49, 0.24, 0.12, 0.06, 0.03 and 0 ng/mL. As one for zero concentration, the "sample dilution solution" is used as that.

### (4) Antigen-antibody reaction -1

To the anti-frog vitellogenin antibody-solid-phased plate whose temperature is returned to room temperature, each of the "sample" and "vitellogenin standard solution" respectively prepared in (2) and (3) is added at 50 µL/well, followed by reacting at room temperature (18 to 25 degrees centigrade) for 60 min.

### (5) Preparation of cleaning liquid

During an antigen-antibody reaction, the "cleaning liquid (concentration factor: 6)" and distilled water are mixed at a ratio of 1: 5 to prepare a "cleaning liquid".

### (6) Removal of unreacted matters-1

A reaction liquid is discarded, and 300 µL/well of the cleaning liquid prepared in (5) is used three-times to wash the inside of the well. After the third cleaning liquid is discarded, a microplate that is turned upside-down is lightly tapped with paper towel or the like to completely remove the cleaning liquid.

### (7) Preparation of biotin-labeled antibody solution

To the "powder of biotin-labeled antibody", 3 mL of 15 mL of the "label dilution solution" is added and dissolved to prepare a "biotin-labeled antibody solution".

### (8) Antigen-antibody reaction-2

An aliquot of 50 µL of the "biotin-labeled antibody solution" prepared in (7) is added to each well, followed by reacting at room temperature (18 to 25 degrees centigrade) for 60 min.

### (9) Removal of unreacted matters-2

A reaction liquid is discarded, and an aliquot of 300 µL of the cleaning liquid prepared in (5) is used three-times to wash the inside of the well. After the third cleaning liquid is discarded, the microplate that is turned upside-down is lightly tapped with paper towel or the like to completely remove the cleaning liquid.

### (10) Preparation of enzyme-labeled streptavidin solution

To the "enzyme-labeled streptavidin", 3 mL of 15 mL of the "label dilution solution" is added and dissolved to prepare an "enzyme-labeled antibody solution".

### (11) Reaction between biotin and streptavidin

An aliquot of 50 µL of the "enzyme-labeled streptavidin solution" prepared in (10) is added to each well, followed by reacting at room temperature (18 to 25 degrees centigrade) for 60 min.

### (12) Removal of unreacted matters-2

A reaction liquid is discarded, and a 300 µL of the cleaning liquid prepared in (5) is used three-times to wash the inside of the well. After the third cleaning liquid is discarded, the microplate is turned upside-down and lightly tapped with paper towel or the like to completely remove the cleaning liquid.

### (13) Preparation chromogenic reagent

The "chromogenic substrate solution" and the "chromogenic substrate dilution solution" are mixed at a ratio of 1: 100 to prepare a "chromogenic reagent".

### (14) Chromogenic reaction/reaction stop

One hundred micro-liters of the "chromogenic reagent" prepared in (13) are added to a well followed by reacting at room temperature for 30 min, further followed by adding 100 µL of the "reaction stop solution" to the well to stop the reaction.

### (15) Colorimetry and calculation of concentration

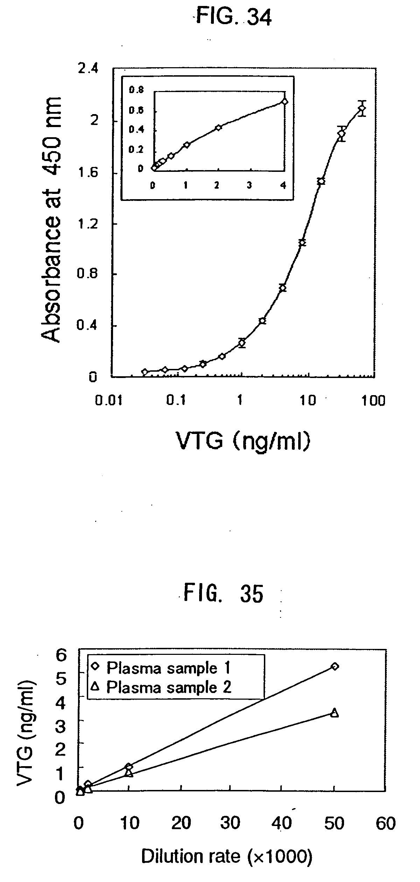

By use of a plate reader, the absorbance at a wavelength 450 nm is measured. A vitellogenin concentration in the sample is calculated from a calibration curve (Fig. 34). Furthermore, by use of a vitellogenin recovery rate curve (the VTG recovery rate versus sample dilution factor), measurements are corrected. It was found that a quantification range according to the procedure was 0.06 to 62.4 ng/ml and the present procedure was 50 times more sensitive than an existing procedure. As shown in Fig. 35, it was found that when the blood plasma was diluted at a factor of 50 or more, the dilution-dependent linear regression could be obtained.

The present invention is not restricted to the abovementioned examples. For instance, in place of the measurement plate in the detection kit, it goes without saying that an immunochromatography method may be used.

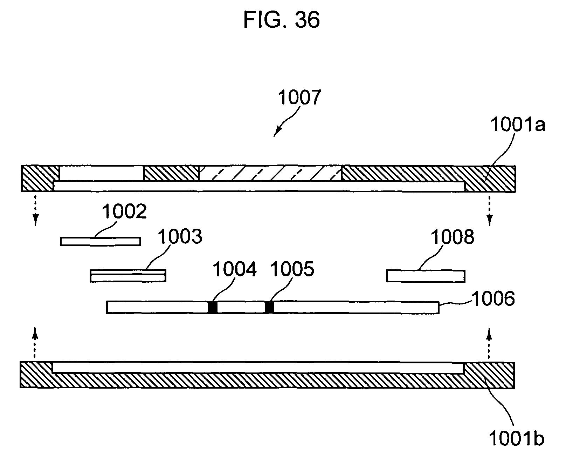

The immunochromatography method uses, as a principle, a sandwich method and is widely used in such as pregnancy determination and so on. Now, an immunochromatography strip 1007 in the present embodiment will be described based on Fig. 36.

As shown in Fig. 36, an immunochromatography strip 1007 has a structure where between plastics covers 1001a and 1001b, a sample pad 1002, a conjugate pad 1003, a membrane 1006 and an absorption pad 1008 are interposed. In the membrane 1006, a judgment line 1004 antibody and a control line 1005 antibody (anti-rabbit IgG antibody) are coated on the respective predetermined places.

A test body, when dropped inside of a region covered with the plastics covers 1001a and 1001b, permeates inside of the sample pad 1002 and the conjugate pad 1003 positioned therebelow and there permeates in a horizontal direction (from left to right in the drawing) while reacting with a gold colloid-labeled Anti-VTG antibody.

The judgment line 1004 has solid-phased antibodies. When the test body passes through there, an antigen-antibody reaction occurs to develop red color, and part of the test body reacts with labeled antibody-specific antibodies solid-phased ahead thereof in the control line 1005 and develops red color. In a positive reaction, two of the judgment line and the control line develop red color and in a negative reaction only one of control line develops red color.

In the next place, a method of manufacturing an immunochromatography device will be described.

On the membrane 1006, judgment line 1004 antibodies (anti-VTG antibodies) and control line 1005 antibodies (anti-rabbit IgG antibodies) are coated in lines, followed by drying to immobilize, further followed by applying blocking to suppress the non-specific adsorption, still further followed by washing, and thereby coating of the antibodies comes to completion. As shown in Fig. 36, when various kinds of members including these are sandwiched with the plastics covers 1001a and 1001b, an immunochromatography device is manufactured.

Subsequently, a specific measurement method that uses the immunochromatography device will be described.

Firstly, a measurement with a vitellogenin sample of a known concentration confirmed to be in the measurement range of 20 ng/ml to 10 mg/ml. In the next place, blood was sampled from an adult male Xenopus laevis exposed to 1 nM E2 for one week, obtained blood serum was diluted in three levels (x 1, x 100 and x 10,000), and 100 µl thereof was dropped in a sample pad 1002. After standing for substantially 15 min, from a dilution level where the reaction became negative, a concentration range of vitellogenin was specified. A concentration range specified according to the present kit, when compared with the concentration quantified according to the ELISA method, exhibited complete coincidence.

### Industrial Applicability

As described above, according to a detection kit, a detection method and an environmental evaluation method according to the present invention, frog vitellogenin can be detected with excellent sensitivity and accuracy. Accordingly, by use thereof, influences of endocrine disruptors in environments can be comprehensively evaluated.

## Claims

1. A detection kit comprising:
a measurement plate having a plate body that has a bottomed well wherein a sample is injected and a primary antibody that is solid-phased on a surface of the well and recognizes a frog vitellogenin;
a standard frog vitellogenin that is injected in the well where the primary antibody is solid-phased; and
a secondary antibody that is injected in the well where the sample or standard frog vitellogenin is injected to recognize the frog vitellogenin.

2. The detection kit according to claim 1, wherein the sample is a frog blood plasma or blood serum.

3. The detection kit according to claim 1, wherein the secondary antibody is labeled with a labeling compound.

4. The detection kit according to claim 1, wherein the primary antibody is adsorbed on the surface of the well and the surface of the well is blocked with a blocking agent.

5. A detection kit comprising:
a first plate that has a bottomed well where a sample and an antibody are injected and mixed, the antibody recognizing a frog vitellogenin and labeled with a labeling compound;
a second plate having a bottomed well in which a mixture liquid of the sample and antibody is injected; and
a standard frog vitellogenin that is solid-phased as an antigen on a surface of the well of the second plate.

6. The detection kit according to claim 5, wherein the sample is a frog blood plasma or blood serum.

7. The detection kit according to claim 5, wherein the antigen is solid-phased on the surface of the well of the second plate and blocked with a blocking agent.

8. A measurement plate comprising:
a plate body that has a bottomed well wherein a sample is injected; and
a primary antibody that is solid-phased on a surface of the well and recognizes a frog vitellogenin.

9. A measurement plate comprising:
a plate body that has a bottomed well where a mixture of a sample and an antibody is injected, the antibody recognizing a frog vitellogenin and labeled with a labeling compound; and
a frog vitellogenin that is solid-phased as an antigen on a surface of the well of the plate.

10. A detection method to detect a frog vitellogenin with a detection kit according to claims 1-6 or 7.

11. A detection method comprising the steps of:
reacting a sample and a primary antibody that recognizes a vitellogenin contained in the sample; and
reacting a complex and a secondary antibody, the complex compounded of the vitellogenin and the primary antibody, the secondary antibody recognizing the vitellogenin.

12. The detection method according to claim 11, wherein the secondary antibody is labeled with a labeling compound.

13. The detection method according to claim 11 or 12, further comprising the step of:
directly or indirectly reacting the secondary antibody bonded with the complex and a chromogenic reagent to measure based on a coloring reaction thereof an amount of vitellogenin in the test body.

14. A detection method comprising the steps of:
reacting a sample and a primary antibody that is labeled with a labeling compound and recognizes a vitellogenin contained in the sample to obtain a complex; and
competitively reacting the complex and a vitellogenin.

15. The detection method according to claim 14 further comprising the step of:
reacting a reaction product obtained according to the competitive reaction and a chromogenic reagent to measure based on a coloring reaction therebetween an amount of the vitellogenin in the sample.

16. An evaluation method comprising the steps of:
reacting a sample and a primary antibody that recognizes a vitellogenin contained in the sample;
reacting a secondary antibody that is labeled with a labeling compound and recognizes the vitellogenin with a complex of the vitellogenin contained in the sample and the primary antibody;
reacting a label in the secondary antibody bonded to the complex and a chromogenic reagent to measure an stained amount; and
calculating an amount of the vitellogenin from the stained amount to evaluate based on the amount of the vitellogenin.

17. The environment evaluation method according to claim 16, wherein the sample is a frog blood plasma or blood serum.

18. An evaluation method comprising the steps of:
reacting a sample and an antibody that is labeled with a labeling compound and recognizes a frog vitellogenin contained in the sample to obtain a complex;
causing the complex and vitellogenin to competitively react; and
reacting a reaction product obtained according to the competitive reaction and a chromogenic reagent, calculating based on a coloring reaction thereof an amount of vitellogenin in the test body to evaluate based on the amount of the vitellogenin.

19. The evaluation method according to claim 18, wherein the sample is a frog blood plasma or blood serum.

20. A polyclonal antibody of a frog vitellogenin, produced by the processes of:
immunizing a mammal with a frog vitellogenin as an antigen;
sampling an anti-blood serum from the immunized mammal; and
isolating as an IgG from the anti-blood serum.

21. A manufacturing method of a frog vitellogenin antibody, comprising the steps of:
obtaining an IgG from an anti-blood serum sampled after a mammal is immunized with a frog vitellogenin as an antigen; and
purifying the IgG with an affinity column.

22. The manufacturing method of a frog vitellogenin antibody according to claim 21, wherein the affinity column is bonded with a male frog serum protein.

23. The manufacturing method of a frog vitellogenin antibody according to claim 22, wherein the affinity column is bonded with a frog vitellogenin.

24. An evaluation method comprising the steps of:
cultivating a hepatocyte due to an amphibian;
administering a sample to the hepatocyte; and
detecting a response to the sample of the cultivated hepatocyte.
